# EUROPEAN PATENT APPLICATION

(11) **EP 0 566 410 A2**
(43) Date of publication of application: **20.10.1993**
(21) Application number: 93302935.7
(22) Date of filing: 15.04.1993
(51) Int. Cl.: C12N 15/24, C07K 13/00, A61K 37/02

(54) **Derivatives of adipogenesis inhibitory factor, their preparation and their use**

(30) Priority: 17.04.1992 JP 97567/92; 29.01.1993 JP 14056/93
(71) Applicant: SANKYO COMPANY LIMITED, Chuo-ku, Tokyo 103 (JP)
(72) Inventor: Miyadai, Kenji, c/o Sankyo Company Limited, Tokyo 140 (JP); Kawashima, Ichiro, c/o Sankyo Company Limited, Tokyo 140 (JP); Ohsumi, Jun, c/o Sankyo Company Limited, Tokyo 140 (JP); Takiguchi, Yo, c/o Sankyo Company Limited, Tokyo 140 (JP); Ito, Yasuhiro, c/o Sankyo Company Limited, Tokyo 140 (JP)
(74) Representative: Gibson, Christian John Robert

(57) **Abstract**

The present invention relates to derivatives of adipogenesis inhibitory factor (AGIF) which are equivalent to mature AGIF lacking from 2 to 9 of the N-terminal amino acids, as well as to nucleic acid coding for such derivatives, vectors and host cells containing such nucleic acid and methods for the production of such derivatives. Also claimed is the use of derivatives of AGIF which are equivalent to mature AGIF lacking from 1 to 9 of the N-terminal amino acids as therapeutic agents against cytopenia and morbid obesity.

## Description

The present invention relates to novel derivatives of adipogenesis inhibitory factor (AGIF), as well as to processes for their preparation and the uses thereof. It also relates to the use of certain known derivatives of AGIF.

AGIF is a protein which has been shown to inhibit the differentiation of pre-adipocytes into adipocytes. The protein, which is otherwise known as interleukin 11 (IL-11), has been isolated from the stromal cell line KM-102, which is derived from human bone marrow [Sequence ID No. 4, Kawashima et al. FEBS Letters, 283 (1991) 199-202 and Ohsumi et al. FEBS Letters, 288 (1991) 13-16]. The isolated protein has been shown to inhibit the differentiation of the pre-adipocyte cell line H-1/A, which is derived from mouse bone marrow, into adipocytes.

PCT WO 92/13955, published after the earliest priority date of the present application, describes a fusion protein between thioredoxin and a derivative of IL-11 (AGIF) in which the N-terminal proline residue of the IL-11 is deleted. This disclosure does not discuss either the derivative of AGIF per se, or the activity of this compound.

It is known that all haematopoietic cells, (i.e. cells involved in the formation of blood) for example the erythrocytes, neutrophils, monocytes, eosinophils, basophils, platelets and lymphocytes, are derived from mother cells in the bone marrow by differentiation, also known as haematopoesis. These mother cells are more commonly known as pluripotent stem cells. Hematopoiesis and prolifeiation of pluripotent stem cells are known to be regulated by a variety of haematopoietic factors, as well as by cell-to-cell interaction with bone marrow stromal cells. Examples of such haematopoietic factors are the the colony stimulating factors, for example granulocyte-macrophage colony stimulating factor (GM-CSF), macrophage colony stimulating factor (M-CSF) and granulocyte colony stimulating factor (G-CSF). Haematopoiesis generally takes place in the "haematopoietic microenvironment", as described by Dexter and Spooncer in Ann. Rev. Cell Bioi., 3 (1987) 423-441.

Pre-adipocytes are known to have a support function in the haematopoietic microenvironment but, when these differentiate into adipocytes, this support function is lost [Hiroaki Kodama "SOSHIKI BAIYO (Tissue Culture)", 12 (1986) 191-195]. This has been demonstrated using the mouse pre-adipocyte cell line PA6. This cell line is able to support haematopoesis, but this support function decreases as the PA6 cells differentiate into adipocytes [Kodama et aₗ., J. Cell. Physiol., 118 (1984) 233-240].

It is also known that culturing PA6 cells and mouse bone marrow cells, i.e. pluripotent stem cells, together will result in the formation of blast cell colonies, megakaryocyte colonies and macrophage colonies [Hiroaki Kodama, "JIKKEN IGAKU (Experimental Medicine)", 5 (1987) 826-830]. As AGIF has been isolated from human bone marrow cells, it is believed that this protein acts directly on bone marrow pre-adipocytes to inhibit their differentiation, thereby allowing the pre-adipocytes to support the haematopoesis of the pluripotent bone marrow stem cells.

Experiments have furthermore shown that the pre-adipocyte cell line H-1/A, which is derived from bone marrow, produces colony stimulating factor (CSF), a known haematopoietic factor. The amount of CSF produced by the pre-adipocytes decreases as these differentiate into adipocytes [Nakamura et al., Proc. Soc. Exp. Biol. Med., 179 (1985) 283-287].

It is therefore suggested that, by inhibiting the differentiation of pre-adipocytes into adipocytes, AGIF allows for extended production of CSF by the pre-adipocytes, thereby promoting haematopoesis of pluripotent stem cells [Ohsumi et al. FEBS Letters, 288, (1991) 13-16].

An increase in the haematopoesis of stem cells effectively results in an increase in the production of the cellular elements in blood. It is therefore to be expected that an inhibitor of adipogenesis will have practical value in the treatment of conditions in which the blood cell count is diminished, for whatever reason. Examples of such conditions include cytopenia (i.e. a diminution of the cellular elements in blood or other tissues arising from any disease) and anaemia. In addition, inhibition of adipogenesis will result in a reduction in the number of adipocytes, or fat cells, therefore suggesting that inhibitors of adipogenesis will be of value in the treatment of conditions such as obesity.

In view of the advantages to be gained by inhibiting the differentiation of pre-adipocytes into adipocytes, there is a need for further such inhibitors of adipogenesis, which inhibitors would, preferably, possess the activity of mature AGIF.

The present invention provides, as novel compounds, a human polypeptide selected from a human adipogenesis inhibitory factor derivative lacking from 2 to 9 of the N-terminal amino acids, functionally equivalent derivatives thereof and precursors therefor.

The invention also provides a pharmaceutical composition for the treatment or prophylaxis of a cytopenia, or for the treatment or prophylaxis of morbid obesity, which composition comprises an effective amount of an anti-cytopenia or anti-morbid obesity compound in admixture with a pharmaceutically acceptable carrier or diluent, wherein said anti-cytopenia or anti-morbid obesity compound is a human polypeptide selected from a human adipogenesis inhibitory factor derivative lacking from 1 to 9 of the N-terminal amino acids, functionally equivalent derivatives thereof and precursors therefor.

The invention also provides the use of a human adipogenesis inhibitory factor derivative lacking from 1 to 9 of the N-terminal amino acids, functionally equivalent derivatives thereof and precursors therefor, as a pharmaceutical.

The present invention also provides nucleotide sequences encoding these polypeptides, vectors including such nucleotide sequences and host cells transformed with such nucleotide sequences or vectors, as well as processes for preparing these polypeptides. These further embodiments are described in greater detail hereafter.

The present invention will now be further described with reference, as necessary, to the accompanying drawings, in which:
Figure 1 shows the results of Western blotting of the limited digestion by trypsin of the mature form of AGIF.
Figure 2 shows the biological activity of the culture supernatant of COS-1 cells treated with trypsin.
Figure 3 represents a preparation diagram for the plasmid M13mp19 (20-2)µ containing cDNA encoding mature AGIF [Sequence ID No. 9, Sequence ID No. 10, Sequence ID No. 13].
Figure 4 represents the base sequence in the vicinity of the Stu I restriction site of M13mp19 (20-2)µ [Sequence ID No. 12, Sequence ID No. 13].
Figure 5 is a diagram of the expression plasmid pMAL-c-20-2APro containing the sequence of AGIFΔPro [Sequence ID No. 14, Sequence ID No. 15].
Figure 6 shows a comparison of the DNA sequences contained in M13mp19 (20-2) and M13mp19 (20-2)APro [Sequence ID No. 18, Sequence ID No. 19].

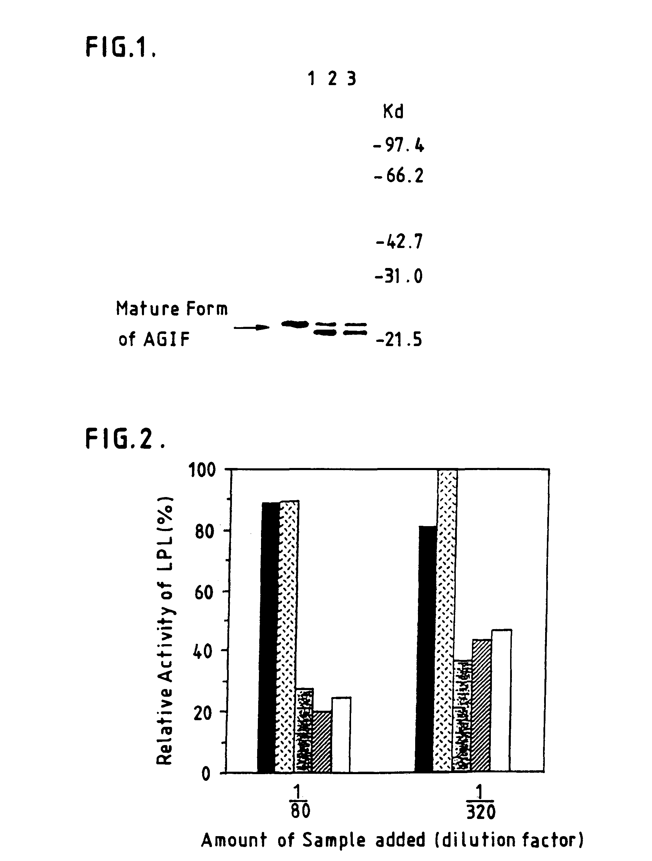

In Figure 1, lane 1 represents the culture supernatant of untreated COS-1 cells, lane 2 represents the culture supernatant of COS-1 cells treated with trypsin, and lane 3 represents the culture supernatant of COS-1 cells in which the reaction was stopped with trypsin inhibitor following treatment with trypsin. The sizes of the molecular weight markers are indicated on the right hand side.

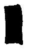 In Figure 2, the solid bar 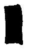 represents the culture supernatant of COS-1 cells transfected with a negative control plasmid (pcDL-SR a296), the dotted bar 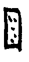 represents that in which trypsin inhibitor vas added to the culture supernatant of COS-1 cells transfected with pcDL-SRa296, the shaded bar 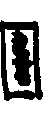 represents the culture supernatant of COS-1 cells transfected with pSR a-20-2 and the diagonally lined bar 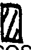 represents the culture supernatant in which trypsin inhibitor was added to the culture supernatant of COS-1 cells transfected with pSR a-20-2. The blank bar 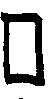 represents the culture supernatant to which trypsin inhibitor was added following trypsin treatment of the culture supernatant of COS-1 cells transfected with pSR a-20-2.

Lipoprotein lipase (LPL) activity was expressed as a percentage, taking the value resulting from the addition of Dulbecco's modified Eagle's medium without sample as 100% activity.

The polypeptide of the present invention is preferably selected from polypeptides containing amino acids 3 to 178 to amino acids 10 to 178 of the following sequence (Sequence ID No. 2): equivalents, mutants and variants thereof, precursors therefor and derivatives thereof.

As illustrated above, the present invention provides derivatives of AGIF, each derivative lacking specific amino acids from the N-terminal of the mature form of AGIF. From the above sequence, it may be seen that the present invention provides derivatives of AGIF having sequences comprising amino acid numbers 3 to 178, 4 to 178, 5 to 178, 6 to 178, 7 to 178, 8 to 178, 9 to 178 and 10 to 178 of the above sequence, as well as equivalents, mutants, derivatives and variants thereof and precursors therefor.

The polypeptides of the present invention thus comprise, in their active form, from 169 to 176 amino acid residues. The polypeptides of the present invention have an approximate molecular weight within the range of 22,000 to 23,000 daltons, when measured using sodium dodecyl sulfate polyacrylamide gel electrophoresis under reducing conditions.

The present invention provides AGIF derivatives, as described above, as well as functionally equivalent derivatives thereof and precursors therefor.

By functionally equivalent derivatives and equivalents, as used herein, is meant any AGIF derivative of the present invention in which one or more of the amino acids of the sequence has been modified, either articificaiiy or naturally, which modified polypeptide still retains at least a substantial part of the biological activity of the unmodified derivative. The biological activity of the polypeptides of the present invention is described in detail hereinafter. In brief, this biological activity includes any one or all of the following: the ability to suppress the lipoprotein lipase activity of adipocytes, the ability to induce the production of colony stimulating factor by pre-adipocytes, the ability to promote the formation of antibody by B cells, the ability to promote the formation of megakaryocyte colonies and platelets and the ability to reduce the Go phase of haematopoietic stem cells.

In general, it will be appreciated that the activity of any given protein is dependent upon certain conserved regions of the molecule, whilst other regions have little importance associated with their particular sequence, and may be virtually or completely redundant. Accordingly, the present invention also includes any variants or mutants on the sequence of the AGIF derivative which still show substantial AGIF-type activity. Such variants and mutants include, for example, deletions, additions, insertions, inversions, repeats and type-substitutions (e.g. substituting one hydrophilic residue for another, except when the residue is strongly hydrophilic or hydrophobic, as a rule). Small changes will generally have little effect on activity, unless they are an essential part of the molecule, and may be a side-product of genetic manipulation. Examples of such small changes include the generation of extra restriction sites, as desired.

It will be appreciated that the coding sequence for the polypeptides of the present invention may be modified in any manner desired, provided that there is no adverse effect on the activity of the resulting polypeptide. Spot mutations and other changes may be effected, for example to assist in genetic manipulation by adding or deleting restriction sites, or otherwise to enhance or modify the molecule.

For example, it is already known that, in many polypeptides, cysteine residues can be converted to serine residues, without a significant loss of activity. This has been demonstrated with respect to interleukin 2 (IL-2) by Wang et al. in Science 224 (1984) 1431-1433.

Variants of the AGIF derivatives of the present invention include naturally occuring AGIF derivatives which share the sequence of mature AGIF lacking from 2 to 9 of the N-terminal amino acid residues, but which vary therefrom in a manner to be expected within a large population. Within this definition lie allelic variations and those peptides from other species showing a similar type of activity and having a related sequence.

Precursors of the AGIF derivatives of the present invention include, for example, those having N-terminal substituents, as well as fusion proteins. Such N-terminal substituents are preferably so chosen as to be cleaved, degraded or otherwise removed prior to, or on, reaching the target site in the body. Such substituents may spontaneously decompose or cleave in the bodily environment, for example due to changes in pH or ionic concentration etc., or they may be actively cleaved by the action of, for example, enzymes, either before or after administration.

Suitable N-terminal substituents include leader sequences and groups such as esters, as well as methionine of formylmethionine residues.

Substituents such as leader sequences, as well as fusion proteins, will generally be a by-product of any expression system used to obtain the AGIF derivative. Such leader sequences or fusion proteins will normally be cleaved in the expression system but may, alternatively, be cleaved in the body, for example at the target site. Such sequences tend to serve no particular function with regard to the activity of the AGIF derivatives, but rather tend to be involved advantageously in the expression of the polypeptide.

In the present invention, where a leader sequence is used, it may be appropriate to use the naturally occuring leader sequence, for example as illustrated by amino acid numbers -21 to 0 in the above sequence (Sequence ID No. 2). However, any suitable sequence may be used, especially where such has been specifically developed for a given expression system.

The AGIF derivative of the present invention may be produced in the form of a fusion protein, as desired, and this is normally as an aid to the expression of the polypeptide. Such fusion proteins are generally cleaved in the expression system, either spontaneously or after addition of, for example, an appropriate enzyme, to result in the production of the AGIF derivative. The choice of fusion partner is not essential to the present invention, but rather tends to depend on the choice of expression system. Thus, for expression in certain prokaryotic cells, such as E. coli, as exemplified hereinafter, it may be appropriate to use maltose binding protein, or a derivative thereof, as a fusion partner for the AGIF derivative of the present invention.

A preferred polypeptide of the present invention is represented by the following sequence (Sequence ID No. 7):
as well as equivalents, mutants, variants and derivatives thereof, and precursors therefor, as hereinabove defined.

The present invention also provides nucleotide sequences encoding all or part of the AGIF derivatives of the present invention. Such sequences may be either DNA or RNA and they may be produced by techniques standard in the art, for example: by reverse-engineering of a polypeptide sequence as provided herein; by chemical sythesis using the phosphite triester method [Hunkapiller et al. Nature 310 (1984) 105-111]; or by the synthesis of cDNA, using the enzyme reverse transcriptase, from an RNA template prepared from cells expressing mature AGIF, followed by selection and amplification of the cDNA by the polymerase chain reaction (PCR), using primers forthe sense and anti-sense strands prepared using the cDNAsequence. The cDNAsequence of the mature AGIF is described in the literature [Kawashima et al. FEBS Letters 283 (1991) 199-202]. The person skilled in the art would be able to use this published cDNAsequence in, for example, the production of oligonucleotide primers for use in the polymerase chain reaction, in order to obtain sequences suitable for use in the present invention.

It will be appreciated that any one given reverse-engineered sequence will not necessarily hybridise well, or at all, with any given complementary sequence reverse-engineered from the same peptide, owing to the degeneracy of the genetic code. This is a factor common in the calculations of those skilled in the art, and the degeneracy of any given sequence is frequently so broad as to make it extremely difficult to synthesise even a short complementary oligonucleotide sequence to serve as a probe for the naturally occurring oligonucleotide sequence.

The degeneracy of the code is such that, for example, there may be four, or more, possible codons for frequently occurring amino acids. Accordingly, therefore, it can be seen that the number of possible coding sequences for any given peptide can increase exponentially with the number of residues in that peptide. As such, it will be appreciated that the number of posssible coding sequences forthe AGIF derivatives of the present invention may be extremely large and there may be little to chose between the sequences. However, certain factors may need to be taken into account which may affect the choice of coding sequence, for example the choice of expression system and the frequency of codon usage by that system. It may also be desirable to balance the G+C content of the sequence according to the expression system concerned.

Selection of codons when constructing a nucleotide sequence can be performed arbitrarily, to a certain degree, and suitable codons can be selected using standard methods, such as those described by Grantham et al. in Nucleic Acids Res. 9 (1981) r43-r47. Consideration should be taken of, for example, the frequency of codon usages in a particular host.

A preferred coding sequence is selected from nucleotides numbers 87 to 614 to nucleotides numbers 108 to 614 of the following sequence (Sequence ID No. 1):
as well as mutants, variants and complementary sequences thereof.

The present invention therefore includes nucleotide sequences which include nucleotide numbers 87 to 614, 90 to 614, 93 to 614, 96 to 614, 99 to 614, 102 to 614, 105 to 614 and 108 to 614 of the above sequence.

The terms "mutant" and "variant" as used above have similar meanings to those used in connection with the peptide sequence mutatis mutandis. Alteration of the nucleotides in the chosen coding sequences can be performed using standard techniques such as, for example, site specific mutagenesis. This technique, which is described by Mark et al. in P.N.A.S. 81 (1984) 5662-5666, involves the use of a primer composed of synthesised oligonucleotides coding for the desired alteration.

It will be appreciated that, whilst a mutant or variant of a peptide sequence will always be reflected in the coding nucleotide sequence, the reverse is not necessarily true. Accordingly, it may be possible for the nucleotide sequence to be substantially changed (for example as described above with regard to the degeneracy of the genetic code) without affecting the peptide sequence in any way. It is known that, in general, the genes of eukaryotes demonstrate such polymorphism, for example as is illustrated by the interferon gene [Nishi et al. J. Biochem. 97 (1985) 153-159]. Polymorphism may also exist, depending on the cell type from which RNA originated. Such mutants and variants are within the scope of the invention.

The present invention also provides nucleotide sequences which hybridise with the sequence shown above (Sequence ID No. 1) and preferably such sequences should show in excess of 50%, more preferably 70% and most preferably 80% homolgy with the sequence shown above.

The nucleotide sequences of the present invention are preferably sequences of DNA. Such sequences may be used alone, for example as probes, but it is generally preferred that they form part of an expression system. Thus, it is preferred that the DNA sequence forms part of a vector useful in an expression system.

The general nature of vectors for use in accordance with the present invention is not essential to the invention. In general, suitable vectors, expression vectors and constructions therefor, will be apparent to those skilled in the art.

Suitable expression vectors may be based on 'phages or plasmids, both of which are generally host specific, although these can often be engineered for other hosts. Other suitable vectors include cosmids and retroviruses, and any other vehicles, which may or may not be specific for a given system. Control sequences, such as recognition, promoter, operator, inducer, terminator and other sequences essential and/or useful in the regulation or expression, will be readily apparent to those skilled in the art, and may be associated with the natural AGIF sequence or with the vector used, or may be derived from any other source as suitable. The vectors may be modified or engineered in any suitable manner.

A particularly preferred nucleotide sequence, coding for an AGIF derivative of the present invention, is as follows: (Sequence ID No. 7):
and mutants, variants and complementary sequences thereof, for example as hereinabove defined.

The present invention also provides a method for the production of a polypeptide as hereinabove described, which process comprises production of an expression vehicle containing a DNA sequence encoding an AGIF derivative, as hereinabove described, transfection of an appropriate host cell with said vector, culture of said host cell under conditions which enable expression of said AGIF derivative, optionally treating the culture with an enzyme capable of cleaving the expressed AGIF derivative at a specific site, and isolation of said expressed AGIF derivative from the culture. This method allows the production of the desired AGIF derivative on an industrial scale, achieving both high yield and high purity.

In general, there are a number of methods which can be used to produce the peptide and nucleotide sequences of the present invention.

In one embodiment, the naturally occurring peptide may be produced in a conventional manner, and digested to remove the desired number of amino acids from the N-terminal. Such digestion may be facilitated by alteration of the nucleotide sequence to provide a site, such as a thermo- or cold -labile site, for example, or a recognisable residue or sequence in the encoded peptide, whereat chemical cleavage, for example, or enzyme cleavage, may be effected. Suitable peptidases are well known to those skilled in the art and the use of trypsin is otherwise appropriate.

One suitable method for the preparation of the polypeptides of the present invention is to prepare DNA encoding the mature form of AGIF using the methods described in the literature [Kawashima et al. FEBS Letters 283 (1991) 199-202]. This DNA is then subjected to techniques such as in vitro mutagenesis in order to arrive at a DNA sequence encoding the desired polypeptide, i.e. a polypeptide which is equivalent to mature AGIF, but which is lacking from 2 to 9 of the N-terminal amino acid residues. The resulting sequence may then be expressed directly or, alternatively, fused with a second DNA sequence, such that a fusion protein results upon expression. The fusion protein may then be digested in a standard fashion, for example by use of an appropriate enzyme, to obtain the desired polypeptide. Partners for formation of a fusion protein with the AGIF derivative of the present invention include, for example, any polypeptides which include the recognition sequence at the C-terminal for specific enzyme. One suitable such partner includes the maltose binding protein or derivatives thereof. Upon formation of a fusion protein between maltose binding protein and the AGIF derivative of the present invention and, after addition of the blood coagulation factor, Xa, the fusion protein is cleaved, and the desired AGIF derivative results.

DNA coding for the AGIF derivative of the present invention may be incorporated into a suitable vector, and host cells of prokaryotes or eukaryotes may then be transformed with such a vector. Using techniques standard in the art for construction of a vector and expression of a protein in a host cell, the DNA encoding the AGIF derivative may be expressed in the host cells.

Cultures useful for the production of the AGIF derivatives of the present invention may suitably be cultures of any living cells, and may vary from prokaryotic expression systems up to eukaryotic expression systems.

Preferred prokaryotic hosts include, for example Escherichia coli and Bacillus subtilis. In order to express the gene of interest in these host cells, the host cells are transformed with a vehicle containing the DNA sequence to be expressed, for example a plasmid vector. This vector will normally contain a replicon, that is an origin of replication derived from a species having compatibility with the host, and regulator sequences. It is furthermore desirable for the vector to contain a sequence imparting selectivity to the expression character, or phenotype, of the transformed cells.

The choice of vector and/or host cell is not essential to the present invention and this choice will, as discussed above, depend on various factors. We generally find that, when using E. coli as the host cell, the publicly available K12 strain is suitable, whilst the publicly available plasmids pBR322 and pUC are suitable as the vectors.

As discussed hereinabove, it is often necessary, or at least desirable, to include various control sequences when expressing a protein such as the AGIF derivatives of the present invention. When using E. coli as the host cell, suitable promoters include the tryptophan (trp) promoter, the lactose (lac) promoter, the tryptophan- lactose (tac) promoter, the lipoprotein (Ipp) promoter, the bacteriophage-originating lambda (k) P_{L} promoter and the polypeptide chain extending factor Tu (tufB) promoter. Any one of these promoters may be used in production of the AGIF derivatives of the present invention.

When using B. subtilis as host cell, we generally prefer to use the publicly available strain 207-25. Asuitable vector for use with this host cell is pTUB228, described by Ohmura et al. in J. Biochem. 95 (1984) 87-93. The regulator sequence of the X-amylase gene of B. subtilis is frequently used as the promoter in this vector. If expression outside the cell is desired, i.e. if the polypeptide is to be secreted out of the cell, a DNA sequence coding forthe signal sequence of a-amylase can be linked to the coding sequence of the AGIF derivative using standard techniques.

Eukaryotic cells which may be used to express the polypeptides of the present invention include the cells of vertebrates, insects, yeasts, etc.. As hereinabove described, the choice of cell is not essential to the present invention. Generally, however, COS cells, which are derived from monkeys [Gluzman, Cell 23 (1981) 175-182], and the dihydrofolate reductase deficient strain of Chinese hamster ovarian cells (CHO) [Urlaub and Chasin, P.N.A.S. USA, 77 (1980) 4216-4220] are the most frequently used for expression of proteins in vertebrate cells, and we have found that these are particularly suitable for use in connection with the present invention.

For expression in a vertebrate cell it is generally desirable to include standard regulatory sequences in the expression vector, such as a promoter sequence located upstream from the gene to be expressed, an RNA splicing site, a polyadenylation site, a transcription termination sequence, etc. This vector may also have an origin of replication, as necessary. Atypical example of such an expression vector is pSV2dhfr, which has the early promoter of SV40 (Subramani et aₗ., Mol. Cell. Biol. 1 (1981) 854-864).

Yeast cells are also typically used as the eukaryotic microorganism host. The Saccharomyces species, such as Saccharomyces cerevisiae, are particularly favoured as expression hosts. Many vectors are known which are suitable for expression of proteins in yeast cells. It is preferred that such vectors also include regulatory sequences. For the present invention, it is preferred to use the promoter of the alcohol dehydrogenase gene [Bennetzen and Hall, J. Biol. Chem. 257 (1982) 3018-3025] orthe promoterofthe acid phosphatase gene [Miyanohara et al., P.N.A.S. USA, 80 (1983) 1-5].

In the process of the present invention, it is particularly preferred to express the polypeptides in COS cells. The vector containing the DNA sequence to be expressed then typically contains an SV40 origin of replication (which enables autonomous replication of the vector in COS cells) as well as a transcription promoter, a transcription termination signal and an RNAsplicing site. Such sequences are standard in the art, and appropriate sequences should be apparent to the man skilled in the art.

The expression vector can be introduced into the host cells using techniques which are standard in the art. Thus, for example, an expression vector can be introduced into COS cells using the diethylaminoethyl- dextran (DEAE-dextran) method, as described by Luthman and Magnusson in Nucleic Acids Res. 11 (1983) 1295-1308, the calcium phosphate-DNA coprecipitation method, as described by Graham and van der Ed in Virology, 52 (1973) 456-457, or the electropuncture or electroporation method described by Neumann et al. in EMBO J., 1 (1982) 841-845. For introducing an expression vector into CHO cells, such that the transformed cells can stably produce the AGIF derivative, suitable techniques include co-transfection of a vector capable of expressing a neo gene functioning as a G418 resistant marker, such as pRSVneo [Sambrook et al., "Molecular Cloning - A Laboratory Manual", Cold Spring Harbor Laboratory, NY (1989)] or pSV2-neo (Southern and Berg, J. Mol. Appl. Genet., (1982) 327-341), together with the expression vector, followed by selection of G418 resistant colonies.

Depending on the host cell chosen, the expressed AGIF derivative will be retained inside the cell or secreted out of the cell into the culture medium. The choice of medium for culturing and the culture conditions are not essential to the present invention but, rather, are dependent on the host cell selected. Suitable media are well known in the art, and will be readily apparent to the skilled person. In the present invention, we generally find that the desired polypeptide is expressed sufficiently in COS cells when the medium is either RPMI-1640 medium or Dulbecco's modified Eagle's medium (DMEM) to which a serum component, such as foetal bovine serum (FBS), has been added as necessary.

The polypeptide of the present invention may be isolated and purified from the cells or the culture medium using any one or combination of a variety of known isolation procedures, taking advantage of, for example, the physical and chemical properties of the protein. Specific examples of such methods include treatment by ordinary protein precipitants, ultrafiltration, various types of chromatography, such as molecular sieve chromatography (gel filtration), adsorption chromatography, ion exchange chromatography, affinity chromatography and high performance liquid chromatography (HPLC), dialysis or combinations of any one of these, or other, methods.

The present invention furthermore provides the use of the AGIF derivates hereinabove described as pharmaceuticals. In addition, the present invention provides the new pharmaceutical use of the known AGIF derivative, which is the mature form of AGIF lacking the N-terminal amino acid.

Accordingly, the present invention provides the use as a pharmaceutical of a human polypeptide selected from a human adipogenesis inhibitory factor derivative lacking from 1 to 9 of the N-terminal amino acids, functionally equivalent derivatives thereof and precursors therefor.

More particularly, the present invention provides the use as a pharmaceutical of a human polypeptide selected from polypeptides containing amino acids 2 to 178 to amino acids 10 to 178 of the following sequence (Sequence ID No. 2):

as well as equivalents, mutants and variants thereof, precursors therefor and derivatives thereof as hereinabove defined.

The preferred polypeptides for the use hereinabove described have one of the following two sequences:
as well as equivalents, mutants and variants thereof, precursors therefor and derivatives thereof, such as are hereinabove defined.

The AGIF derivatives of the present invention and, in particular, those derivatives hereinabove exemplified, have a wide variety of activities. These activities may be summarised as follows:
1. Suppression of lipoprotein lipase activity of adipocytes.
2. Induction of production of colony stimulating factor (CSF) by pre-adipocytes.
3. Promotion of antibody formation by B cells.
4. Promotion of formation of megakaryocyte colonies.
5. Promotion of the production of platelets.
6. An ability to reduce the Go phase of haematopoietic stem cells.

In view of the above activities, the AGIF derivatives of the present invention are expected to be of great value as therapeutic agents. In particular, they are expected to be of value in the treatment of cytopenias, such as thrombocytopenia and leukopenia, in the enhancement of the function of the immune system, in the prevention or alleviation of morbid obesity, in the treatment and diagnosis of various anaemias, such as aplastic anaemia, and other blood diseases and in the treatment and diagnosis of infections caused by viruses, bacteria and parasites. It is also anticipated that the use of the AGIF derivatives of the present invention will allow savings in blood component transfusions.

As used herein, "cytopenia" should be taken to cover a diminution of the cellular elements in blood or other tissues arising from any disease, such as thrombocytopenia, refractory pancytopenia, leukopenia caused by toxins or radiation, cytopenia following bone marrow transplants or cytopenia following chemotherapy caused by carcinostatics, etc., as well as arising from any immune disease which may occur incidentally to such conditions.

"AGIF activity" as used herein includes any one or more of the activities listed under numbers 1 to 6 hereinabove.

The activity of theAGIF derivatives of the present invention, that is polypeptides equivalent to mature AGIF but lacking from 1 to 9 of the N-terminal amino acids, is illustrated by the following Examples.

The polypeptides of the present invention are derived from compounds found naturally in the body and, as such, they have low levels of toxicity. When administered to male adult mice of the strain DDY (with a body weight of approximately 20 g), and after observation for 5 days, the polypeptides of the present invention were shown to have no appreciable toxicity at dosages of 500 mg/kg body weight or less.

In view of this activity, the AGIF derivatives of the present invention, that is polypeptides equivalent to mature AGIF but lacking from 1 to 9 of the N-terminal amino acids, are suitable for use as a therapeutic agent for cytopenia and morbid obesity.

The AGIF derivatives of the present invention, that is polypeptides equivalent to mature AGIF but lacking from 1 to 9 of the N-terminal amino acids, can be used either alone or in combined administration with other therapeutic drugs.

When the AGIF derivatives of the present invention are used to treat morbid obesity, the composition may be used alone or in combination with other suitable anti-obesity drugs, examples of which include appetite suppressors, parenteral absorption suppressors, digestive enzyme inhibitors, metabolism accelerating hormones, lipid synthesis inhibitors and insulin secretion suppressors. Furthermore, the composition may also be used in combination with dietary and/or exercise therapy. In this case, the composition is able to enhance the activity or effects of treatment with other anti-obesity drugs, as well as enhance the effects of treatment with non drug related anti-obesity therapy.

When the AGIF derivatives of the present invention are used to treat cytopenias, the composition may be so prepared as to contain, or be used in combination with, other suitable haematopoietic factors, examples of which include IL-1 to IL-10, leukaemia inhibitory factor (LIF), stem cell factor, GM-CSF, G-CSF, M-CSF, megakaryocyte colony stimulating factor (Meg-CSF), tumor necrosis factor (TNF), interferon (IFN) and erythropoietin (EPO). Combined administration, either in the form of separate compositions or in the form of a combined composition, results in an enhancement of the activity of the haematopoietic factor by the polypeptide of the present invention.

The polypeptides of the present invention may be administered by any route commonly used for compounds having this type of activity and may be formulated in admixture with conventional additives or adjuvants for this purpose. For example, for oral administration, they may be formulated as tablets, capsules, granules, powders or syrups; whilst for parenteral administration, they may be formulated as injections, intravenous drip infusions or suppositories.

These pharmaceutical preparations can be prepared by any conventional means using such additives as vehicles, binders, disintegrators, lubricants, stabilizers and corrigents.

When being prepared for administration by injection or intravenous drip infusion, the pharmaceutical preparation should be in the form of a parenterally acceptable aqueous solution, i.e. a solution that does not contain any pyrogens. Such solutions can be prepared by any conventional means, taking into consideration such factors as pH, isotonicity and stability, and is within the ambit of a person skilled in the art.

Although the dosage may vary, depending on, for example, the age, sex, body weight, diet, symptoms and severity of infection of the patient, as well as the period of administration and otherfactors of clinical effect. For an adult human patient, however, a suitable daily dosage may be from 0.01 to 1000 mg/kg body weight per day, which may be administered as a single dose or divided into several doses. For parenteral administration, a suitable daily dose ranges between 0.01 and 100 mg/kg body weight, administered either by subcutaneous injection, intramuscular injection or intravenous injection.

The present invention may be further described with reference to the following non-limiting Examples, in which Examples A to D describe various methods for determining the activity of the polypeptides of the present invention, and Examples 1 to 4 describe methods for the preparation of the polypeptides of the present invention.

In the following Examples, 3T3-L1 cells, which are derived from mouse embryonic fibroblasts and are as described by Green and Kehinde in Cell, (1974) 113-116, were purchased from the American Cell Type Culture Collection (ATCC). Where reference is made in the following Examples to the culture of 3T3-L1 cells, these cells were cultured at 37°C in a humidified gaseous mixture of 10% (v/v) C0₂ and 90% (v/v) air. Subculturing of the cells was performed in medium A.

### Medium A:

Dulbecco's modified Eagle's medium (DMEM) containing 4.5 g/I of glucose, (manufactured by Gibco);
10% (v/v) immobilized foetal bovine serum (FBS) (manufactured by Hyclone); and
10 mM N-2-hydroxyethylpiperazine-N'-ethane sulfonic acid (HEPES) (pH 7.2, manufactured by Sigma).
Differentiation of 3T3-L1 cells into adipocytes was induced according to the method of Rubin et al. in J. Biol. Chem., 253 (1978) 7570-7578.

### EXAMPLE A

### Inhibition of the Transformation of 3T3-L Cells into Adipocytes

3T3-L1 cells were cultured by suspension of the cells in medium A, as defined above, at a density of 1.0 x 10⁴ cells/ml. 0.5 ml of this culture medium was pipetted into each well of a 48-well multicluster dish (manufactured by Costar), and the cells were cultured until they reached a state of confluence, i.e. for approximately 3 days. The medium was then replaced with fresh medium A, and the cells were cultured for a further 2 days. At the end of this time, the medium was replaced by an equivalent amount of medium B, a medium for inducing adipogenic differentiation.

### Medium B:

DMEM containing 4.5 g/I of glucose;
10 mM HEPES (pH 7.2);
3% (v/v) immobilized FBS;
5 µg/ml of bovine insulin (manufactured by Sigma);
8 µg/ml of d-biotin (manufactured by Sigma);
4 µg/ml of pantothenic acid (manufactured by Sigma);
1.0 µM dexamethasone (manufactured by Sigma); and
0.5 mM isobutylmethylxanthine (manufactured by Aldrich).

The sample of material to be tested was added to the wells at the same time as the medium B. The sample was added in a small amount to each well, this amount being less than one tenth of the amount of medium B used.

After the addition of medium B and the sample, the cells were cultured further, with replacement of the medium B with fresh medium B every 2 days. Fresh sample was also added each time the medium was replaced. Four to seven days after the first addition of medium B and the sample, the medium was replaced with an equivalent amount of medium C, a medium for maintaining adipocytes.

### Medium C:

DMEM containing 4.5 g/I of glucose;
5% (v/v) immobilized FBS;
10 mM HEPES (pH 7.2); and
100 ng/ml of bovine insulin.

The cells were cultured for 2 days in medium C, after which they were fixed with 5% v/v formaldehyde. The lipid droplets that had accumulated within the cells and the cell nuclei were stained with oil red O and haematoxylin, respectively, according to the methods described by Mitomo and Takayama ["RINSHOKENSA-KOZA (Clinical Testing Seminar)", Vol. 12, "Pathology" (1982), Ishiyaku Publishing]. In particular, for oil red 0 staining, the fixed cells were first washed with distilled water, then washed with a 60% solution of isopropyl alcohol in water after which the cells were stained for 10 minutes with a 0.3% oil red 0 solution in 60% isopropyl alcohol. At the end of this time, the cells were again washed with the isopropyl alcohol solution, and with distilled water.

Microscopic photographs were then taken of the stained cells, and the stained cells were counted. Counts were made both of the cells containing red fat granules, and the cells containing stained nuclei. The rate of adipogenic differentiation was then calculated using the following formula:
Adipogenic Differentiation Rate (%) ₌₁₀₀ₓ x No. of Cells Containing Fat Droplets No. of Nucleated Cells

### EXAMPLE B

### Suppression of Lipoprotein Lipase Activity

The ability of a sample to suppress lipoprotein lipase (LPL) activity may be measured using known methods. In the following, the cells were prepared and tested following the method described by Beutler et al. in J. Immunol., 135 (1985) 3972-3977. Briefly, this was performed as follows.

Differentiated 3T3-L1 adipocytes were prepared using the method described in Example A, above. However, medium B was used alone, i.e. no sample was added to the differentiating cells at'that stage. After growth in medium B, this medium was replaced with fresh medium C, having the composition shown in Example A, above. The sample to be tested was also added at this stage, at an amount of approximately one eightieth of the volume of the medium, and the cells were cultured for 18 hours (see Figure 2). At the end of this time, the medium was removed and the cells were washed twice with PBS(-) buffer (phosphate buffered saline without Mg²⁺ and Ca2+, manufactured by Nissui Seiyaku). 300 µl of medium D were then added to each well.

### Medium D:

DMEM containing 4.5 g/I glucose;
5% (v/v) immobilized FBS;
10 mM HEPES (pH 7.2);
100 ng/ml of bovine insulin; and
10 U/ml sodium heparin (manufactured by Novo Industries).

The cells were then cultured for 1 hour, after which 100 µl of the culture supernatant was collected and used for measurement of activity. Measurements were made in triplicate for each sample, and the final result was obtained by determining the average of the three measurements.

Lipoprotein lipase activity was measured using the method of Nilsson-Ehle and Schotz described in J. Lipid Res., 17 (1976) 536-541. This method may be summarised as follows. 100 µl of the culture supernatant, prepared as described above, were mixed with an equal volume of a substrate solution having the following composition:
13 mM glycerol-tri[9,10(n)-3H]oleic acid (51.8 KBeq/µmol, manufactured by Amersham);
1.3 mg/ml L-a-phosphatidylcholine distearoyl (manufactured by Sigma);
20 mg/ml bovine serum albumin (manufactured by Sigma);
135 mM Tris-hydrochloride (Tris-HCI, pH 8.1, manufactured by Sigma);
16.5% (v/v) glycerol; and
16.5% (v/v) immobilized FBS.

13 mM glycerol-tri[9,10(n)-³H]oleic acid (51.8 KBeq/µmol) was prepared by diluting glycerol-tri[9,-10(n)-³H]oleic acid (370 GBeq/mmol) (manufactured by Amersham) with triolein (manufactured by Sigma), followed by purification using silica gel column chromatography.

The mixture was allowed to react at 37°C for 120 minutes. At the end of this time, the reaction was stopped by the addition of 1.05 ml of 0.1 M potassium carbonate-boric acid buffer (pH 10.5) and 3.25 ml of a 141:125:100 by volume mixture of methanol, chloroform and heptane. After vigorous stirring, the reaction mixture was centrifuged at 3,000 x g for 15 minutes. The ³H count of the water-methanol layer was then measured using a liquid scintillation counter. 1 unit of lipoprotein lipase activity was defined as the amount of activity producing 1 µmol of fatty acid in 1 minute.

### EXAMPLE C

### Increase in Platelet Count

750 µg AGIFΔPro, prepared as described in Examples 3 and 4, were dissolved in 3.75 ml PBS(-) buffer containing 0.1% (w/v) bovine serum albumin.

Seven-week old male Sprague-Dawley rats (Nippon SLC Co. Ltd.) were purchased, maintained for a week before use in the experiments, and placed, at random, into two groups of 5 rats each. One group was selected for treatment with AGIFΔPro, and the other group was designated as a control group. The rats in the treatment group received 100µg of AGIF ΔPro per kg weight subcutaneously. The concentration of the solution of AGIFAPro was adjusted according to the weight of individual rats, so that the volume of the solution administered remained at 1 ml. The rats in the control group were given 1 ml of the vehicle buffer only. The rats in both groups were treated once a day for a period of 5 days, and they were each weighed before each treatment.

At the end of the treatment period, the blood was sampled, and the platelet count determined. Twenty four hours after the final administration, the rats were anesthetized with ether, and syringes containing 0.5 ml of a 3.8% trisodium citrate dihydrate solution were used to collect 4.5 ml of blood by cardiac puncture. The numbers of platelets, white blood cells and red blood cells in the various samples were determined straight away, using a Coulter counter (Model T5 / 40, manufactured by Coulter Electric Inc.). The results are shown below. Treatment group Platelet Count = 1,123,000 ± 66,500 Control group Platelet Count = 893,000 ± 23,700

The counts are given as the average ± standard error, per cubic millimeter of sample.

The counts obtained for the treatment and control groups were compared using the multiple comparison method described by Dunnett et al. in Biometrics, September (1964) 482-489. As a result of this comparison, it was determined that the platelet count in the treatment group, i.e. that group receiving AGIFΔPro, was significantly greater than that in the control group (with a probability of less than 5%).

There was no significant difference in the count of red and white blood cells from the rats in the two groups. In addition, the rats in each group gained weight at a similar rate during the period of the test.

AGIFΔPro has also been shown to increase the platelet count in normal mice.

Furthermore, the administration of 200 wg/kg of AGIFAPro over a 10 day period to rats treated with the carcinostatic agent Carboplatin accelerated the recovery from thrombocytopenia caused by treatment with the Carboplatin.

### EXAMPLE D

The AGIF derivative prepared as described in Example 2, i.e. the derivative in which the 9 N-terminal amino acids of the mature protein are missing, was tested in the method described in Example C, above. This derivative similarly shows the ability to increase the platelet count.

### EXAMPLE 1

### Expression of AGIF

### (a) Construction of an Expression Vector for AGIF

The method of Kawashima et al., described in FEBS Letters, 283 (1991) 199-202, was followed to produce the plasmid pcD-20-2. In accordance with this method, a cDNA library was prepared in the expression vector pcD from poly(A) ⁺RNA isolated from the stromal cell line KM-102. Stromal cell line KM-102 is derived from human bone marrow. The library was screened with an oligonucleotide selective for AGIF, and appropriate clones were selected. After further testing of these clones (for example using the test method described in Example A, above), the plasmid from the clone which was capable of directing expression of a protein having AGIF-type activity in COS-1 cells (a publicly available cell line derived from monkey cells) was selected and designated pcD-20-2. The cDNAcoding forAGIFwas then extracted from the pcD-20-2 clone using techniques standard in the art. This cDNAwas then incorporated into the high expression vector pcDL-SR a296 [described by Takebe et al. in Mol. Cell. Biol., 8 (1988) 466-472] using techniques described by Ohusmi et al., in FEBS Letters, 288 (1991) 13-16. The resulting vector, designated pSR a-20-2, directs a high level of expression of the AGIF polypeptide in cells such as COS-1 cells.

### (b) Expression of AGIF in COS-1 Cells

The plasmid pSR a-20-2, prepared as described in step (a), was then transfected into COS-1 cells. Transfection was performed by electroporation using a gene introduction unit, GTE-1, available from Shimadzu Seisakusho Ltd. The COS-1 cells were prepared for transfection by growth of the cells in a flask [cells were grown in Dulbecco's modified Eagle's medium containing 10% fetal bovine serum, for 3 days and at 37°C] until the culture reached a state of semi-confluence. The cells were then collected from the flask by treatment with trypsin-ethylenediaminetetraacetic acid (trypsin-EDTA), after which the collected cells were washed twice with phosphate buffered saline (-) [PBS(-)] (manufactured by Nissui Seiyaku). Afterthis, the washed cells were suspended in PBS(-) buffer at a concentration of 1 x 10⁸ cells/ml. The plasmid DNA, i.e. the pSR a-20-2 plasmid prepared as described in step (a), was prepared for transfection by treatment according to the caesium chloride method, after which the solution containing the treated plamids was adjusted to 200 µg/ml by the addition of PBS(-) buffer.

For the transfection, 20 µl of the COS-1 cell suspension, prepared as described above, and 20 µl of the plasmid solution, prepared as described above, were mixed, and the mixture was then placed in the chamber of an FCT-13 electroporation device (manufactured by Shimadzu Seisakusho Ltd.) in which the distance between electrodes is 2 mm. Two batches of pulses of 600V/50 µsec were then applied to the device, with an interval of 1 second between the pulse batches. Then, the mixture from the chamber of the electroporation device was added to a solution of 20 ml of Dulbecco's modified Eagle's medium containing 10% v/v fetal bovine serum, after which the resulting mixture was transferred to a Petri dish (diameter: 150 mm). This was then cultured overnight in an atmosphere containing 5% C0₂ by volume, whilst maintaining the temperature at 37°C. At the end of this time, the culture supernatant was removed by aspiration, the cells and Petri dish were washed with serum-free Dulbecco's modified Eagle's medium, and 20 ml of Dulbecco's modified Eagle's medium were added to the washed cells. The cells were then cultured under the same conditions for a further 3 days.

At the end of this time, the culture supernatant was removed from the culture and was tested, by Western blotanalysis, to confirm the presence of AGIF [Sequence ID No.4]. The antibodyAGIFp15, which was prepared as described by Ohsumi et al. in FEBS Letters 288 (1991) 13-16, was used in this Western blot.

### EXAMPLE 2

### Preparation of a polypeptide having AGIF activity

0.5 µg of trypsin (manufactured by Sigma) were added to 500 µl of the COS-1 cell culture supernatant containing AGIF prepared according to step (b) of Example 1, and the resulting mixture was incubated at 37°C for20 minutes. At the end of this time, 0.5 µg of lima bean trypsin inhibitor (manufactured by Sigma) was added to the mixture to inactivate the trypsin. The solution was then analysed by Western blot, and the results of this Western blot analysis are shown in Figure 1. As indicated in Figure 1, the mature form of AGIF demonstrates an apparent molecular weight of approximately 23,000 daltons, as determined by sodium dodecyl sulfate polyacrylamide gel electrophoresis (SDS-PAGE) under reducing conditions. After treatment of this mature AGIF with trypsin, a polypeptide demonstrating a molecular weight of approximately 22,000 daltons, as determined by sodium dodecyl sulfate polyacrylamide gel electrophoresis under reducing conditions, was formed. Analysis of the trypsin induced digestion of the mature AGIF using a densitometer (CS-930, manufactured by Shimadzu Seisakusho Ltd.) indicated that approximately 60% of the mature form of AGIF was digested by trypsin into a polypeptide having a molecular weight, as determined by sodium dodecyl sulfate polyacrylamide gel electrophoresis under reducing conditions, of approximately 22,000 daltons.

It is known that trypsin typically demonstrates substrate specificity, i.e. that it "recognises" certain amino acids. In particular, trypsin will only cut the peptide bond on the carboxy side of either of the amino acids arginine or lysine in a polypeptide. It was therefore hypothesised that the lower molecular weight derivative of mature AGIF was produced as a result of the hydrolysis by trypsin of the bond between the arginine residue at position 9 from the N-terminal of mature AGIF, and the amino acid residue at position 10 from the N-terminal.

In order to substantiate this theory, a variant of mature AGIF was prepared in which the arginine residue at position 9 from the N-terminal was replaced with another amino acid (but not lysine), for example with asparagine. This mutant was prepared using an in vitro Mutagenesis System (manufactured by Amersham). Once prepared, the mutant was treated with trypsin under the same conditions as discussed above. After treatment with this enzyme, there was no reduction in the molecular weight of the polypeptide and, therefore, no digestion. This confirms the fact that the AGIF derivative, i.e. the polypeptide having a molecular weight of approximately 22,000 daltons, as determined by sodium dodecyl sulfate polyacrylamide gel electrophoresis under reducing conditions, which was obtained as a result of trypsin treatment of the mature form of AGIF, had valine as its N-terminal amino acid, i.e. the 10th amino acid from the N-terminal of the mature form of AGIF.

After the culture supernatant obtained by transfection of COS-1 cells with pSR a-20-2 had been treated with trypsin, as described above, the trypsin was inactivated by the addition of a lima bean trypsin inhibitor (manufactured by Sigma) to a final concentration of 1 wg/ml. As indicated in Figure 1, this solution contains approximately 60% of a polypeptide having a molecular weight of approximately 22,000 daltons, as determined by sodium dodecyl sulfate polyacrylamide gel electrophoresis under reducing conditions.

The ability of this solution to suppress the activity of lipoprotein lipase (LPL) was measured, using the techniques described in Example B, above. The results of this test are illustrated in Figure 2. By these tests, it was demonstrated that the ability of the trypsin treated protein solution to suppress the activity of LPL was equivalent to that demonstrated by the mature AGIF. In addition to this activity, it has been demonstrated (using techniques as described hereinbefore) that the trypsin treated protein solution suppresses the transformation of mouse embryonic fibroblast 3T3-L1 cells into adipocytes, as well as suppressing the differentiation of the mouse bone marrow pre-adipocyte cell line H-1/A into adipocytes [demonstrated using the method described by Nakamura et al., Proc. Soc. Exp. Biol. Med., 179 (1985) 283-287].

The trypsin treated protein solution, prepared as described above, was separated into two fractions using an ultrafiltration membrane (Centricon C-10, manufactured by Amicon) having a fractional molecular weight of approximately 10,000 daltons, i.e. into a fraction containing polypeptides with a molecular weight less than 10,000 daltons (the membrane-permeable fraction) and a fraction containing polypeptides with a molecuiar weight of more than 10,000 daltons (the membrane-impermeable fraction). The two fractions obtained were tested in order to determine the ability of the fractions to suppress LPL activity in 3T3-L1 adipocytes. The results of these tests indicated that the AGIF-type activity was only present in the membrane-impermeable fraction, i.e. that fraction containing proteins of molecular weight greater than 10,000 daltons. This indicated that AGIF-type activity was present in the polypetide having a molecular weight of approximately 22,000 daltons, formed as a result of trypsin treatment of the mature form of AGIF, and that this activity was not present in the peptide comprising the 9 amino acids removed by the trypsin treatment from the N-terminal of mature AGIF.

### EXAMPLE 3

### Expression of a polypeptide having AGIF activity in Escherichia coli

The following Example shows the preparation and expression of an AGIF derivative which corresponds to mature AGIF minus the N-terminal amino acid.

Vector M13 mp19 RF1 DNA (manufactured by Toyobo), that is double stranded DNA, was digested with the restriction enzyme Bam HI, after which the digested DNAwas treated with alkaline phosphatase to remove the terminal phosphate groups.

Plasmid pSR a-20-2, prepared as described in Example 1, was digested with the restriction enzymes Bam HI and Bgl II. Three fragments resulted from this digestion, and the fragment of approximately 750 base pairs, containing AGIF cDNA, was isolated and purified. The fragment containing the AGIF cDNA was then ligated with the digested M13 mp19 plasmid, using T4 DNA ligase. The resulting DNAwas then used to transform the DH5aF' strain of Escherichia coli (purchased from GIBCO/BRL Life Technologies Inc.) to prepare recombinant phage plaques. 12 clones were selected at random from the resulting plaques, after which, for each clone, single stranded DNAand double stranded RF (replicative form) DNAwere isolated and purified, using conventional techniques. The RF DNA from each of the 12 clones was digested with the restriction enzyme Sma I, and the clone designated M13 mp19 (20-2), whose Sma I digest contained a fragment of approximately 750 base pairs, i.e. containing the AGIF cDNA, was selected.

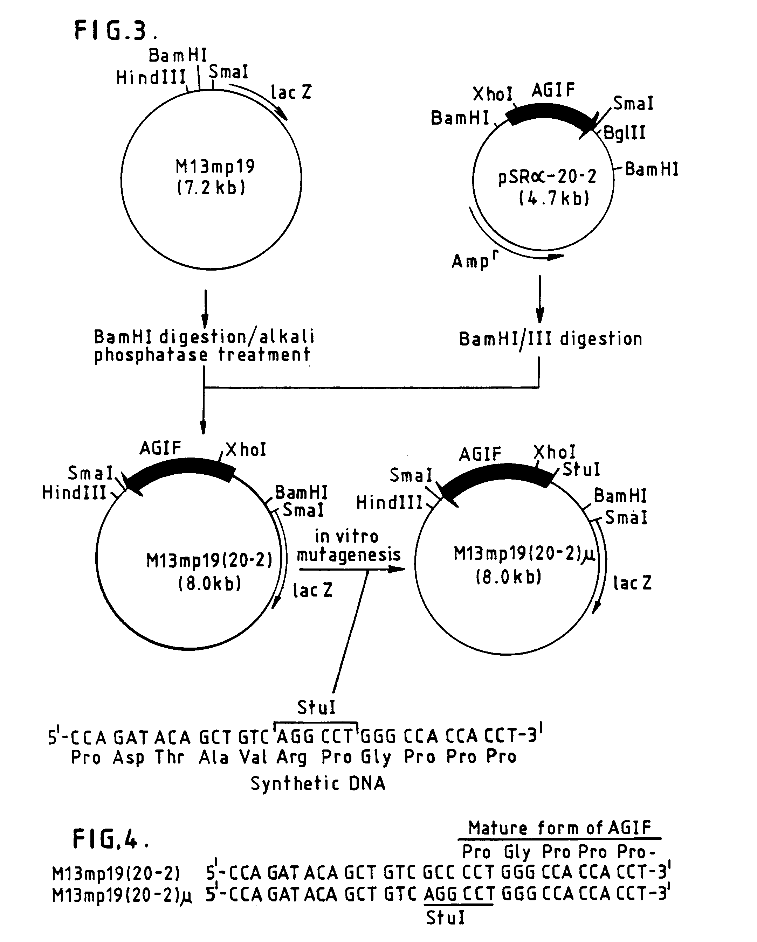

An oligonucleotide, having the following DNA sequence:
(Sequence ID No. 13) was synthesised, using an in vitro mutagenesis system (manufactured by Amersham) and following the manufacturer's recommended procedure, and this oligonucleotide was annealed to the single stranded DNA of the clone M13 mp19 (20-2), (prepared as described above) thereby producing the mutant phage clone designated M13 mp19 (20-2)µ (Figure 3). This procedure allows a restriction site forthe restriction enzyme Stu I (site: AGGCCT) to be introduced into the DNA, and also allows for the DNA encoding mature AGIF [Sequence ID No. 3] to be prepared from the RF DNA of this mutant clone. The Stu I site is introduced at the start of the coding sequence for the mature AGIF polypeptide.

DNA formed into a straight chain by digestion of the E. coli expression vector pMAL-C (manufactured by New England Biolabs) with the restriction enzymes Stu I and Hind III, and a fragment containing AGIF cDNA prepared by digestion of the double stranded RF DNA of M13 mp19 (20-2)µ (prepared as described above) also with Stu I and Hind III, were ligated using T4 DNA ligase. Vector pMAL-C was chosen because it contains a structural gene (malE) coding for maltose binding protein (MBP) downstream from the promoter. E. coli strain TB1 was transformed with this DNA to obtain ampicillin-resistant colonies able to grow in L agar medium containing ampicillin (100 µg/ml).

### L agar:

in distilled water:
1 % Bactotryptone
0.5% yeast extract
1 % NaCI; and
1.5% Bactoagar.

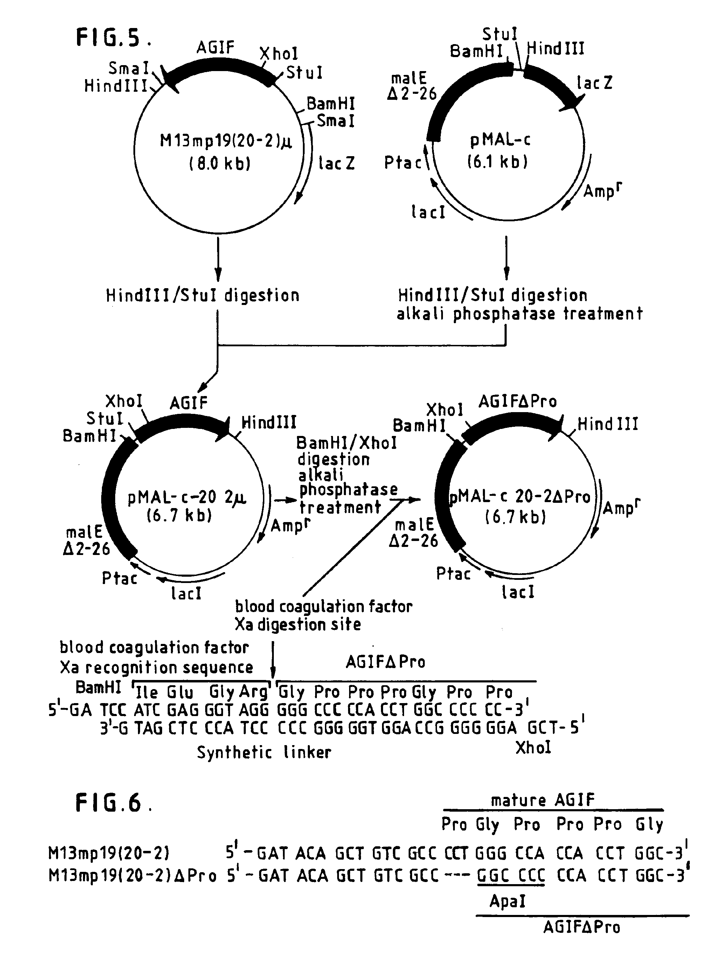

12 clones were selected at random from the resulting colonies and a plasmid clone, designated pMAL-c-20-2µ, shown to contain AGIF cDNAby restriction enzyme mapping, was selected (Figure 5) [Sequence ID No. 14, Sequence ID No. 15].

After this, two types of mutually complementary oligonucleotides were synthesised. These oligonucleotides consisted of a nucleotide sequence coding for mature AGIF (but in which the triplet of bases encoding the N-terminal proline residue of mature AGIF were not included: Sequence ID No. 5) as well as a nucleotide sequence coding for an amino acid sequence recognised by the blood coagulation factor Xa. These oligonucleotides were then annealed to prepare a linker DNA having a Bam HI restriction site at one end, and a Xho I restriction site at the other end (Figure 5). This synthetic DNA linker was then ligated with vector DNA, obtained by digesting the plasmid pMAL-c-20-2µ prepared as described above, with Bam HI and Xho I, and the resulting vector was used to transform the TB1 strain of E. coli. The clone having the target linker was then selected from the resulting ampicillin-resistant colonies, and the plasmid contained in the selected clone was named pMAL-c-20-2APro (Figure 5).

TB1 strain containing pMAL-c-20-2ΔPro was inoculated into 50 ml of medium E.

### Medium E:

in distilled water:
1 % bactotryptone;
0.5% yeast extract;
0.5% NaCI; and
0.2% glucose.

The inoculated medium was then cultured overnight at 30°C, whilst shaking. At the end of this time, 10 ml of the culture liquid was inoculated into 1 liter of fresh medium E, and further cultured with shaking at 30°C. When the absorbance of the culture liquid at 600 nm had reached 0.5, isopropyl β-D-thiogalactopyranoside was added to a final concentration of 0.3 mM, after which the inoculated medium was cultured overnight at 30°C.

At the end of the culture period, the bacteria were collected by centrifugation and suspended in 50 ml of buffer A.

### Buffer A:

in distilled water:
10 mM Tris(hydroxymethyl)aminomethane-HCI (Tris-HCI);
0.2 M NaCI;
1 mM sodium azide;
10 mM 2-mercaptoethanol; and
1 mM ethylenediaminotetraacetic acid (EDTA), pH 7.4.

The resulting suspension was treated ultrasonically for 2 minutes to crush the bacteria, after which the crushed bacteria were separated by centrifugation at 9,000 x g and 4°C for 30 minutes. The supernatant was then collected and diluted with fourtimes its volume of buffer A, after which the diluted supernatant was filtered with a Milex GV filter (manufactured by Millipore) having a pore size of 0.22 µm. The filtered solution was collected and applied to an amylose resin column, with a bed volume of 10 ml, and the column was washed with approximately 80 ml of buffer A. The fusion protein consisting of maltose binding protein and AGIF lacking the N-terminal proline (AGIFΔPro), was eluted from the column using buffer A further containing 10 mM maltose, and approximately 2 ml of the peak fraction were collected.

One unit (1 µg) of blood coagulation factor Xa was added to 20 µl of the crude fraction of the MBP-AGIFΔ-Pro fusion protein (containing about 90 µg of protein), prepared as described above, and the mixture was left to digest at room temperature for 2 to 24 hours. The reaction was followed by sodium dodecyl sulfate polyacrylamide gel electrophoresis under reducing conditions and using a gel containing 12.5% (w/v) of acrylamide. A band corresponding to a polypeptide of approximately 62,000 daltons molecular weight, assumed to be the MBP-AGIFΔPro fusion protein, and a further band, corresponding to a polypeptide of approximately 23,000 daltons molecular weight, and assumed to be AGIFΔPro were observed initially. Over the course of the digestion reaction, the 62,000 dalton band decreased in intensity, whilst the 23,000 dalton band increased in intensity. Western blot analysis of the polypeptide of the 23,000 dalton band indicated that this band reacted with AGIFp15 antibody.

40 units (40 µg) of blood coagulation factorXa were added to 800 µl of the crude fraction of MBP-AGIFΔPro fusion protein (containing approximately 3.6 mg of protein), prepared as described above, and the mixture was left overnight at room temperature. At the end of this time, the reaction mixture was concentrated by trichloroacetic acid (TCA) precipitation treatment, after which it was subjected to sodium dodecyl sulfate polyacrylamide gel electrophoresis, using a gel containing 12.5% (w/v) of acrylamide, under reducing conditions. After this electrophoresis, the protein band was transferred from the polyacrylamide gel to a polyvinylidene difluoride (PVDF) membrane (trade name Immobilon, manufactured by Millipore) using a gel membrane transfer device (KS-8441, manufactured by Marisol) in a transfer buffer [0.02% SDS, 20% methanol and 25 mM Tris-boric acid (pH 9.5)], run at 0.8 mA/cm² at 4°C for a period of 15 hours. After the transfer, the membrane was washed for 5 minutes in 10 mM sodium borate buffer containing 25 mM NaCI, and then for 5 minutes in pure water, whilst shaking. It was then dried. After this, the portion of the membrane to which an approximately 23,000 dalton molecular weight band had been transferred was cut out, and the sequence from the N-terminal to the 9th amino acid residue was determined using a gaseous phase protein sequencer (Model 475A, manufactured by Applied Biosystems). The phenylthiohydantoin (PTH) -amino acids obtained for each reaction cycle were separated and identified by reverse phase high performance liquid chromatography (HPLC) (the chromatography apparatus used was the Model 120A, manufactured by Applied Biosystems). The amino acid sequence determined in this manner was as indicated below
(in which Xaa represents an unidentified amino acid residue. Seq ID No. 11).

Based on this result, it was confirmed that the isolated protein is AGIFΔPro lacking the proline residue from the N-terminal of the mature form of AGIF.

Before biological testing could be performed, a digestion reaction was carried out using blood coagulation factor Xa on the crude fraction of MBP-AGIFΔPro fusion protein, produced according to the method described above. AGIFΔPro produced as a result of this digestion was then isolated by purification of the crude sample. The techniques used include a combination of gel filtration column chromatography and CM Toyopearl Pack 650M (manufactured by Tosoh) column chromatography. When subjected to sodium dodecyl sulfate polyacrylamide gel electrophoresis using a gel containing 12.5% (w/v) acrylamide, and under reducing conditions, the protein in the sample migrated to a position equivalent to a molecular weight of approximately 23,000 daltons. The biological activity of the AGIFΔPro prepared in this manner was then determined. It was confirmed that the protein reacted with the antibody AGIFp15 using Western blot analysis, and that the protein purified in this manner also demonstrated the ability to suppress the differentiation of pre-adipocytes to adipocytes in mouse 3T3-L1 cells. Furthermore, this protein also, surprisingly, suppressed the lipoprotein lipase (LPL) activity of 3T3-L1 cells which had already differentiated into adipocytes. Furthermore, the specific activity of this lipoprotein lipase (LPL) suppression was substantially equal to that of known mature AGIF, as described in PCT Publication No. WO 92/08735.

### EXAMPLE 4

### Expression of a polypeptide having AGIF activity in COS-1 cells

The following Example shows the preparation and expression of an AGIF derivative which corresponds to mature AGIF minus the N-terminal amino acid.

Following procedures similar to those described in Example 3, but using a synthetic oligonucleotide sequence designated PRO-DEL1, having the following sequence:
(Sequence ID No. 16) and single stranded vector M13mp19 (20-2) DNA (prepared as described in Example 3) the mutant phage clone designated M13 mp19 (20-2)ΔPro was produced. This procedure allows the introduction of a restriction site for the restriction enzyme Apa I (site: GGGCCC) into the DNA, and also allows for the DNA encoding AGIFΔPro to be prepared from the RF DNA of this mutant clone. The Apa I site is introduced at the start of the coding sequence for the AGIFAPro polypeptide. The mutant clone was selected by restriction mapping for the Apa I site present in the sequence of PRO-DEL1. Nucleotide sequence analysis revealed that this M13mpl9 (20-2)APro contained the DNA coding for AGIFΔPro, i.e. mature AGIF without the N-terminal proline residue (Figure 6).

Linear vector DNA of approximately 4.5 kb in length lacking a 190 base pair fragment including the N-terminal region of mature AGIF, was prepared by cleavage of plasmid pSRa-20-2, a COS-1-cell expression plasmid for mature AGIF (prepared as described in Example 1) by the restriction enzyme Bal I. The double-stranded RF DNA of M13mpl9 (20-2)APro, prepared as described above, was similarly digested with Bal I. A 187 base pairfragment, including the N-terminal region of AGIF ΔPro, was removed by Bal digestion of the cloning vector, and this fragment was ligated into the linear vector prepared as described above. The resulting DNA was introduced into E. coli DH5a, and ampicillin-resistant colonies that could grow on L-agar plates containing ampicillin at a concentration of 100 µg/ml were selected. 20 clones were selected at random, and each plasmid from these 20 clones was extracted and purified. The clone containing the 187 base pair Bal fragment ligated in the same orientation as the AGIF cDNA in plasmid pSRa-20-2 was selected by analyzing the Apa I site of each plasmid DNA. As a result, one clone was obtained, and the plasmid contained in this clone was designated pSRa-(20-2)APro.

pSRa-(20-2)APro, prepared as described above, is an expression plasmid for AGIFΔPro. This plasmid was transfected into COS-1 cells using techniques similar to those described in Example 1, and AGIFΔPro was produced and released into the culture medium. One liter of serum-free culture supernatant of COS-1 cells transfected with pSRa-(20-2)APro was prepared in this way. After dialysis of the culture supernatant with a 20-fold volume of dialysis buffer [10 mM boric acid-NaOH (pH 9.0) and 13 mM KCI] at 4°C for 15 hours, weak cation exchange chromatography was performed using a Fast Protein Liquid Chromatography (FPLC) system (manufactured by Pharmacia). The chromatography conditions were as follows:
Column: CM-Toyopearl Pack 650M (2.2 X 20 cm, manufactured by Tosoh)
Elution buffer:
Solution A: IOmM boric acid-NaOH(pH 9.0), 13mM KCI
Solution B: Solution A containing 300 mM NaCl
Flow rate : 3 ml/min
Fraction volume: 3 ml/tube
Concentration gradient: Linear concentration gradient from 100% solution A to 100% solution B (for 50 minutes).

The fractions obtained from the chromatography and containing AGIFΔPro were identified by Western blotting, using the known antibody AGIFp15. Three consecutive fractions which were shown by this method to contain the most AGIFΔPro were pooled, concentrated by trichloroacetic acid precipitation, and then analyzed by sodium dodecyl sulfate polyacrylamide gel electrophoresis using a gel containing 12.5% (w/v) acrylamide, and under reducing conditions. After completion of the electrophoresis, the protein bands from the polyacrylamide gel were blotted onto a polyvinylidene difluoride membrane (Pro Blott, manufactured by Applied Biosystems) run at 18 V/cm, at 4°C for 2.5 hours. Blotting was carried out using a gel membrane blotter (KS-8441, manufactured by Marisol) in a transfer buffer [0.02% SDS, 20% methanol and 25mM Tris-borate (pH 9.5)]. After washing of the blotted membrane for 5 minutes with 10 mM sodium borate buffer (pH 8.0) containing 25 mM NaCI, and then for 5 minutes with distilled water, whilst agitating, the membrane was air-dried. After this, the portion of the membrane to which AGIFΔPro had transferred (i.e. the portion equivalent to a molecular weight of approximately 23,000 daltons) was cut out of this membrane, and the sequence of the six N-terminal amino acids of the protein was determined using a gas phase protein sequencer (Model 475A, manufactured by Applied Biosystems).

The phenylthiohydantoin amino acids (PTH-amino acids) obtained in the reaction cycles of the protein se- quencerwere separated and identified by reverse phase high performance liquid chromatography (HPLC) using a Model 120Asystem (manufactured by Applied Biosystems). The amino acid sequence determined by this procedure is as shown below:

(Sequence ID No. 17) This sequence is identical to the sequence of amino acids 2 to 7 of mature AGIF, and it was thereby confirmed that the protein isolated is mature AGIF lacking the N-terminal proline residue.

AGIFΔPro was purified from the serum-free conditioned medium of COS-1 cells transfected with pSRa-(20-2)APro using similar methods to those described for the purification of mature AGIF by Ohsumi et al., FEBS Letters 288 (1991) 13-16. Testing of the product produced in this manner demonstrated that the protein was able to suppress the activity of lipoprotein lipase in 3T3-L1 cells which had already differentiated into adipocytes. The specific activity of the protein in this test was substantially the same as that of mature AGIF in the same test, as described in PCT Publication No. WO 92/08735.

## Claims

1. A polypeptide selected from a human adipogenesis inhibitory factor derivative lacking from 2 to 9 of the N-terminal amino acids, functionally equivalent derivatives thereof and precursors therefor.

2. A polypeptide according to Claim 1, selected from polypeptides containing amino acids 3 to 178 to amino acids 10 to 178 of the following sequence (Sequence ID No. 2):
equivalents, mutants and variants thereof, precursors therefor and derivatives thereof.

3. A polypeptide according to Claim 1 having the following sequence (Sequence ID No. 8):
equivalents, mutants and variants thereof, precursors therefor and derivatives thereof.

4. A polypeptide according to Claim 1, in which the N-terminal amino acid is methionine or N-formylmethionine.

5. A polypeptide according to Claim 1, in which the mutants are peptides having mutations comprising any mutation selected from the group consisting of:
deletions, additions, inversions, insertions, replacements of amino acid residues in the sequence or any combination of said mutations, provided that said mutations do not adversely affect the physiological activity of the polypeptide.

6. A polypeptide according to Claim 1, in which the variants include allelic variants.

7. A polypeptide according to Claim 1, in the form of a fusion protein.

8. A nucleotide sequence encoding a polypeptide selected from a human adipogenesis inhibitory factor derivative lacking from 2 to 9 of the N-terminal amino acids, functionally equivalent derivatives thereof and precursors therefor.

9. A nucleotide sequence according to Claim 8, encoding a polypeptide selected from polypeptides containing amino acids 3 to 178 to amino acids 10 to 178 of the following sequence (Sequence ID No. 2):
equivalents, mutants and variants thereof, precursors therefor and derivatives thereof.

10. A nucleotide sequence according to Claim 8, which includes nucleotides selected from nucleotides numbers 87 to 614 to nucleotides numbers 108 to 614 of the following sequence (Sequence ID No. 1):
and mutants, variants and complementary sequences thereof.

11. A nucleotide sequence according to Claim 8 and having the following sequence (Sequence ID No. 7):
and mutants, variants and complementary sequences thereof.

12. A nucleotide sequence complementary to the sequence defined in Claim 8.

13. A nucleotide sequence according to Claim 8, in which the functionaly equivalent derivatives comprise allelic variants.

14. A nucleotide sequence according to Claim 8, in which the functionally equivalent derivatives include polynucleotides having mutations selected from the group consisting of: deletions, additions, insertions, inversions, replacements of residues in the sequence, or any combination thereof, provided that the mutations do not adversely affect the functional activity of the human adipogenesis inhibitory factor derivative.

15. A nucleotide sequence according to Claim 8, in functional association with at least one control sequence therefor.

16. A nucleotide sequence according to Claim 8 in functional association with at least one sequence selected from promoter sequences, operator sequences, terminator sequences, inducer sequences, or a combination thereof.

17. A vector comprising a nucleotide sequence as defined in any one of Claims 8 to 16.

18. A vector according to Claim 17, which is pSRa-(20-2)APro.

19. A vector according to Claim 17, which is pSRa-20-2.

20. A host cell transformed with a nucleotide sequence as defined in any one of Claims 8 to 16, or with a vector as defined in any one of Claims 17 to 19.

21. A pharmaceutical composition for the treatment or prophylaxis of a cytopenia, or of morbid obesity, which composition comprises an effective amount of an anti-cytopenia compound, or of an anti-morbid obesity compound, in admixture with a pharmaceutically acceptable diluent or carrier, wherein said anti-cytopenia compound, or said anti-morbid obesity compound, is selected from a human adipogenesis inhibitory factor derivative lacking from 1 to 9 of the N-terminal amino acids, functionally equivalent derivatives thereof and precursors therefor.

22. A composition according to Claim 21, wherein said anti-cytopenia compound, or said anti-morbid obesity compound, is a polypeptide selected from polypeptides containing amino acids 2 to 178 to amino acids 10 to 178 of the following sequence (Sequence ID No. 2):
equivalents, mutants and variants thereof, precursors therefor and derivatives thereof.

23. A composition according to Claim 22, wherein said anti-cytopenia compound, or said anti-morbid obesity compound, is a polypeptide having the following sequence (Sequence ID No. 8):
equivalents, mutants and variants thereof, precursors therefor and derivatives thereof.

24. A composition according to Claim 22, wherein said anti-cytopenia compound, or said anti-morbid obesity compound, is a polypeptide of the following sequence (Sequence ID No. 6):
equivalents, mutants and variants thereof, precursors therefor and derivatives thereof.

25. The use of a polypeptide as defined in any one of Claims 21 to 24, as a pharmaceutical.

26. The use of a polypeptide as defined in any one of Claims 21 to 24 in the manufacture of a medicament for the treatment of a cytopenia or for the treatment of morbid obesity.

27. A process for the preparation of a polypeptide as defined in any one of Claims 1 to 7, which process comprises producing an expression vehicle containing a DNA sequence encoding an AGIF derivative, transfection of an appropriate host cell with said vector, culture of said host cell under conditions which enable expression of said AGIF derivative, optionally treating the culture with an enzyme capable of cleaving the expressed AGIF derivative at a specific site, and isolation of said expressed AGIF derivative from the culture.

28. A process according to Claim 27, in which the culture is treated with an enzyme capable of selectively digesting the expressed AGIF derivative prior to isolation of said AGIF derivative.


### SEQUENCE LISTING

(1) GENERAL INFORMATION:
   (i) APPLICANT:
      (A) NAME: SANKYO COMPANY, LIMITED
      (B) STREET: 2-58, HIROMACHI 1-CHOME
      (C) CITY: SHINAGAWA-KU, TOKYO
      (E) COUNTRY: JAPAN
      (F) POSTAL CODE (ZIP): 140
      (G) TELEPHONE: 03(3492)3131
      (H) TELEFAX: 03(3492)3754
   (ii) TITLE OF INVENTION: Derivatives of Adipogenesis Inhibitory Factor, Their Preparation and Their Use
   (iii) NUMBER OF SEQUENCES: 19
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) PRIOR APPLICATION DATA:
      (A) APPLICATION NUMBER: JP 04/097567
      (B) FILING DATE: 17-APR-1992
   (vi) PRIOR APPLICATION DATA:
      (A) APPLICATION NUMBER: JP 05/014056
      (B) FILING DATE: 29-JAN-1993
(2) INFORMATION FOR SEQ ID NO:1:
   (i) SEQUENCE CHARACTERISTICS:
      (A) LENGTH: 1065 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: NO
   (iii) ANTI-SENSE: NO
   (ix) FEATURE:
      (A) NAME/KEY: CDS
      (B) LOCATION: 18..614
   (ix) FEATURE:
      (A) NAME/KEY: mat peptide
      (B) LOCATION: 81..614
   (ix) FEATURE:
      (A) NAME/KEY: sig peptide
      (B) LOCATION: 18..80
   (xi) SEQUENCE DESCRIPTION: SEQ ID NO:1:
   CCTGGCCCTG TGGGGAC ATG AAC TGT GTT TGC CGC CTG GTC CTG GTC GTG        50
                      Met Asn Cys Val Cys Arg Leu Val Leu Val Val
                      -21 -20                 -15
   CTG AGC CTG TGG CCA GAT ACA GCT GTC GCC CCT GGG CCA CCA CCT GGC       98
   Leu Ser Leu Trp Pro Asp Thr Ala Val Ala Pro Gly Pro Pro Pro Gly
   -10                 -5                  1                   5
   CCC CCT CGA GTT TCC CCA GAC CCT CGG GCC GAG CTG GAC AGC ACC GTG      146
   Pro Pro Arg Val Ser Pro Asp Pro Arg Ala Glu Leu Asp Ser Thr Val
               10                  15                  20
   CTC CTG ACC CGC TCT CTC CTG GCG GAC ACG CGG CAG CTG GCT GCA CAG      194
   Leu Leu Thr Arg Ser Leu Leu Ala Asp Thr Arg Gln Leu Ala Ala Gln
           25                  30                  35
   CTG AGG GAC AAA TTC CCA GCT GAC GGG GAC CAC AAC CTG GAT TCC CTG      242
   Leu Arg Asp Lys Phe Pro Ala Asp Gly Asp His Asn Leu Asp Ser Leu
       40                  45                  50
   CCC ACC CTG GCC ATG AGT GCG GGG GCA CTG GGA GCT CTA CAG CTC CCA      290
   Pro Thr Leu Ala Met Ser Ala Gly Ala Leu Gly Ala Leu Gln Leu Pro
   55                  60                  65                  70
   GGT GTG CTG ACA AGG CTG CGA GCG GAC CTA CTG TCC TAC CTG CGG CAC      338
   Gly Val Leu Thr Arg Leu Arg Ala Asp Leu Leu Ser Tyr Leu Arg His
                   75                  80                  85
   GTG CAG TGG CTG CGC CGG GCA GGT GGC TCT TCC CTG AAG ACC CTG GAG      386
   Val Gln Trp Leu Arg Arg Ala Gly Gly Ser Ser Leu Lys Thr Leu Glu
               90                  95                  100
   CCC GAG CTG GGC ACC CTG CAG GCC CGA CTG GAC CGG CTG CTG CGC CGG      434
   Pro Glu Leu Gly Thr Leu Gln Ala Arg Leu Asp Arg Leu Leu Arg Arg
           105                 110                 115
   CTG CAG CTC CTG ATG TCC CGC CTG GCC CTG CCC CAG CCA CCC CCG GAC      482
   Leu Gln Leu Leu Met Ser Arg Leu Ala Leu Pro Gln Pro Pro Pro Asp
       120                 125                 130
   CCG CCG GCG CCC CCG CTG GCG CCC CCC TCC TCA GCC TGG GGG GGC ATC      530
   Pro Pro Ala Pro Pro Leu Ala Pro Pro Ser Ser Ala Trp Gly Gly Ile
   135                 140                 145                 150
   AGG GCC GCC CAC GCC ATC CTG GGG GGG CTG CAC CTG ACA CTT GAC TGG      578
   Arg Ala Ala His Ala Ile Leu Gly Gly Leu His Leu Thr Leu Asp Trp
                   155                 160                 165
   GCC GTG AGG GGA CTG CTG CTG CTG AAG ACT CGG CTG TGACCCGGGG           624
   Ala Val Arg Gly Leu Leu Leu Leu Lys Thr Arg Leu
               170                 175
   CCCAAAGCCA CCACCGTCCT TCCAAAGCCA GATCTTATTT ATTTATTTAT TTCAGTACTG    684
   GGGGCGAAAC AGCCAGGTGA TCCCCCCGCC ATTATCTCCC CCTAGTTAGA GACAGTCCTT    744
   CCGTGAGGCC TGGGGGACAT CTGTGCCTTA TTTATACTTA TTTATTTCAG GAGCAGGGGT    804
   GGGAGGCAGG TGGACTCCTG GGTCCCCGAG GAGGAGGGGA CTGGGGTCCC GGATTCTTGG    864
   GTCTCCAAGA AGTCTGTCCA CAGACTTCTG CCCTGGCTCT TCCCCATCTA GGCCTGGGCA    924
   GGAACATATA TTATTTATTT AAGCAATTAC TTTTCATGTT GGGGTGGGGA CGGAGGGGAA    984
   AGGGAAGCCT GGGTTTTTGT ACAAAAATGT GAGAAACCTT TGTGAGACAG AGAACAGGGA   1044
   ATTAAATGTG TCATACATAT C                                             1065
(2) INFORMATION FOR SEQ ID NO:2:
   (i) SEQUENCE CHARACTERISTICS:
      (A) LENGTH: 199 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Asn Cys Val Cys Arg Leu Val Leu Val Val Leu Ser Leu Trp Pro
   -21 -20                 -15                 -10
   Asp Thr Ala Val Ala Pro Gly Pro Pro Pro Gly Pro Pro Arg Val Ser
    -5                  1               5                  10
   Pro Asp Pro Arg Ala Glu Leu Asp Ser Thr Val Leu Leu Thr Arg Ser
               15                  20                  25
   Leu Leu Ala Asp Thr Arg Gln Leu Ala Ala Gln Leu Arg Asp Lys Phe
           30                  35                  40
   Pro Ala Asp Gly Asp His Asn Leu Asp Ser Leu Pro Thr Leu Ala Met
       45                  50                  55
   Ser Ala Gly Ala Leu Gly Ala Leu Gln Leu Pro Gly Val Leu Thr Arg
   60                  65                  70                  75
   Leu Arg Ala Asp Leu Leu Ser Tyr Leu Arg His Val Gln Trp Leu Arg
                   80                  85                  90
   Arg Ala Gly Gly Ser Ser Leu Lys Thr Leu Glu Pro Glu Leu Gly Thr
               95                  100                 105
   Leu Gln Ala Arg Leu Asp Arg Leu Leu Arg Arg Leu Gln Leu Leu Met
           110                 115                 120
   Ser Arg Leu Ala Leu Pro Gln Pro Pro Pro Asp Pro Pro Ala Pro Pro
       125                 130                 135
   Leu Ala Pro Pro Ser Ser Ala Trp Gly Gly Ile Arg Ala Ala His Ala
   140                 145                 150                 155
   Ile Leu Gly Gly Leu His Leu Thr Leu Asp Trp Ala Val Arg Gly Leu
                   160                 165                 170
   Leu Leu Leu Lys Thr Arg Leu
               175
(2) INFORMATION FOR SEQ ID NO:3:
   (i) SEQUENCE CHARACTERISTICS:
      (A) LENGTH: 534 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: N
   (iv) ANTI-SENSE: N
   (ix) FEATURE:
      (A) NAME/KEY: CDS
      (B) LOCATION: 1..534
      (D) OTHER INFORMATION:
   (xi) SEQUENCE DESCRIPTION: SEQ ID NO:3:
   CCT GGG CCA CCA CCT GGC CCC CCT CGA GTT TCC CCA GAC CCT CGG GCC        48
   Pro Gly Pro Pro Pro Gly Pro Pro Arg Val Ser Pro Asp Pro Arg Ala
     1               5                  10                  15
   GAG CTG GAC AGC ACC GTG CTC CTG ACC CGC TCT CTC CTG GCG GAC ACG        96
   Glu Leu Asp Ser Thr Val Leu Leu Thr Arg Ser Leu Leu Ala Asp Thr
                20                  25                  30
   CGG CAG CTG GCT GCA CAG CTG AGG GAC AAA TTC CCA GCT GAC GGG GAC       144
   Arg Gln Leu Ala Ala Gln Leu Arg Asp Lys Phe Pro Ala Asp Gly Asp
            35                  40                  45
   CAC AAC CTG GAT TCC CTG CCC ACC CTG GCC ATG AGT GCG GGG GCA CTG       192
   His Asn Leu Asp Ser Leu Pro Thr Leu Ala Met Ser Ala Gly Ala Leu
        50                  55                  60
   GGA GCT CTA CAG CTC CCA GGT GTG CTG ACA AGG CTG CGA GCG GAC CTA      240
   Gly Ala Leu Gln Leu Pro Gly Val Leu Thr Arg Leu Arg Ala Asp Leu
    65                 70                  75                  80
   CTG TCC TAC CTG CGG CAC GTG CAG TGG CTG CGC CGG GCA GGT GGC TCT      288
   Leu Ser Tyr Leu Arg His Val Gln Trp Leu Arg Arg Ala Gly Gly Ser
                   85                  90                  95
   TCC CTG AAG ACC CTG GAG CCC GAG CTG GGC ACC CTG CAG GCC CGA CTG      336
   Ser Leu Lys Thr Leu Glu Pro Glu Leu Gly Thr Leu Gln Ala Arg Leu
               100                 105                 110
   GAC CGG CTG CTG CGC CGG CTG CAG CTC CTG ATG TCC CGC CTG GCC CTG      384
   Asp Arg Leu Leu Arg Arg Leu Gln Leu Leu Met Ser Arg Leu Ala Leu
           115                 120                 125
   CCC CAG CCA CCC CCG GAC CCG CCG GCG CCC CCG CTG GCG CCC CCC TCC      432
   Pro Gln Pro Pro Pro Asp Pro Pro Ala Pro Pro Leu Ala Pro Pro Ser
       130                 135                 140
   TCA GCC TGG GGG GGC ATC AGG GCC GCC CAC GCC ATC CTG GGG GGG CTG      480
   Ser Ala Trp Gly Gly Ile Arg Ala Ala His Ala Ile Leu Gly Gly Leu
   145                 150                 155                 160
   CAC CTG ACA CTT GAC TGG GCC GTG AGG GGA CTG CTG CTG CTG AAG ACT      528
   His Leu Thr Leu Asp Trp Ala Val Arg Gly Leu Leu Leu Leu Lys Thr
                   165                 170                 175
   CGG CTG                                                              534
   Arg Leu
(2) INFORMATION FOR SEQ ID NO:4:
   (i) SEQUENCE CHARACTERISTICS:
      (A) LENGTH: 178 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Pro Gly Pro Pro Pro Gly Pro Pro Arg Val Ser Pro Asp Pro Arg Ala
    1               5                  10                  15
   Glu Leu Asp Ser Thr Val Leu Leu Thr Arg Ser Leu Leu Ala Asp Thr
               20                  25                  30
   Arg Gln Leu Ala Ala Gln Leu Arg Asp Lys Phe Pro Ala Asp Gly Asp
           35                  40                  45
   His Asn Leu Asp Ser Leu Pro Thr Leu Ala Met Ser Ala Gly Ala Leu
       50                  55                  60
   Gly Ala Leu Gln Leu Pro Gly Val Leu Thr Arg Leu Arg Ala Asp Leu
   65                  70                  75                  80
   Leu Ser Tyr Leu Arg His Val Gln Trp Leu Arg Arg Ala Gly Gly Ser
                   85                  90                  95
   Ser Leu Lys Thr Leu Glu Pro Glu Leu Gly Thr Leu Gln Ala Arg Leu
               100                 105                 110
   Asp Arg Leu Leu Arg Arg Leu Gln Leu Leu Met Ser Arg Leu Ala Leu
           115                 120                 125
   Pro Gln Pro Pro Pro Asp Pro Pro Ala Pro Pro Leu Ala Pro Pro Ser
       130                 135                 140
   Ser Ala Trp Gly Gly Ile Arg Ala Ala His Ala Ile Leu Gly Gly Leu
   145                 150                 155                 160
   His Leu Thr Leu Asp Trp Ala Val Arg Gly Leu Leu Leu Leu Lys Thr
                   165                 170                 175
   Arg Leu
(2) INFORMATION FOR SEQ ID NO:5:
   (i) SEQUENCE CHARACTERISTICS:
      (A) LENGTH: 531 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: N
   (iv) ANTI-SENSE: N
   (ix) FEATURE:
      (A) NAME/KEY: CDS
      (B) LOCATION: 1..531
      (D) OTHER INFORMATION:
   (xi) SEQUENCE DESCRIPTION: SEQ ID NO:5:
   GGG CCA CCA CCT GGC CCC CCT CGA GTT TCC CCA GAC CCT CGG GCC GAG      48
   Gly Pro Pro Pro Gly Pro Pro Arg Val Ser Pro Asp Pro Arg Ala Glu
     1               5                  10                  15
   CTG GAC AGC ACC GTG CTC CTG ACC CGC TCT CTC CTG GCG GAC ACG CGG      96
   Leu Asp Ser Thr Val Leu Leu Thr Arg Ser Leu Leu Ala Asp Thr Arg
                20                  25                  30
   CAG CTG GCT GCA CAG CTG AGG GAC AAA TTC CCA GCT GAC GGG GAC CAC     144
   Gln Leu Ala Ala Gln Leu Arg Asp Lys Phe Pro Ala Asp Gly Asp His
            35                  40                  45
   AAC CTG GAT TCC CTG CCC ACC CTG GCC ATG AGT GCG GGG GCA CTG GGA     192
   Asn Leu Asp Ser Leu Pro Thr Leu Ala Met Ser Ala Gly Ala Leu Gly
        50                  55                  60
   GCT CTA CAG CTC CCA GGT GTG CTG ACA AGG CTG CGA GCG GAC CTA CTG      240
   Ala Leu Gln Leu Pro Gly Val Leu Thr Arg Leu Arg Ala Asp Leu Leu
    65                  70                  75                  80
   TCC TAC CTG CGG CAC GTG CAG TGG CTG CGC CGG GCA GGT GGC TCT TCC      288
   Ser Tyr Leu Arg His Val Gln Trp Leu Arg Arg Ala Gly Gly Ser Ser
                    85                  90                  95
   CTG AAG ACC CTG GAG CCC GAG CTG GGC ACC CTG CAG GCC CGA CTG GAC      336
   Leu Lys Thr Leu Glu Pro Glu Leu Gly Thr Leu Gln Ala Arg Leu Asp
               100                 105                 110
   CGG CTG CTG CGC CGG CTG CAG CTC CTG ATG TCC CGC CTG GCC CTG CCC      384
   Arg Leu Leu Arg Arg Leu Gln Leu Leu Met Ser Arg Leu Ala Leu Pro
           115                 120                 125
   CAG CCA CCC CCG GAC CCG CCG GCG CCC CCG CTG GCG CCC CCC TCC TCA      432
   Gln Pro Pro Pro Asp Pro Pro Ala Pro Pro Leu Ala Pro Pro Ser Ser
       130                 135                 140
   GCC TGG GGG GGC ATC AGG GCC GCC CAC GCC ATC CTG GGG GGG CTG CAC      480
   Ala Trp Gly Gly Ile Arg Ala Ala His Ala Ile Leu Gly Gly Leu His
   145                 150                 155                 160
   CTG ACA CTT GAC TGG GCC GTG AGG GGA CTG CTG CTG CTG AAG ACT CGG      528
   Leu Thr Leu Asp Trp Ala Val Arg Gly Leu Leu Leu Leu Lys Thr Arg
                   165                 170                 175
   CTG                                                                  531
   Leu
(2) INFORMATION FOR SEQ ID NO:6:
   (i) SEQUENCE CHARACTERISTICS:
      (A) LENGTH: 177 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Gly Pro Pro Pro Gly Pro Pro Arg Val Ser Pro Asp Pro Arg Ala Glu
     1               5                  10                  15
   Leu Asp Ser Thr Val Leu Leu Thr Arg Ser Leu Leu Ala Asp Thr Arg
                20                  25                  30
   Gln Leu Ala Ala Gln Leu Arg Asp Lys Phe Pro Ala Asp Gly Asp His
            35                  40                  45
   Asn Leu Asp Ser Leu Pro Thr Leu Ala Met Ser Ala Gly Ala Leu Gly
        50                  55                  60
   Ala Leu Gln Leu Pro Gly Val Leu Thr Arg Leu Arg Ala Asp Leu Leu
    65                  70                  75                  80
   Ser Tyr Leu Arg His Val Gln Trp Leu Arg Arg Ala Gly Gly Ser Ser
                    85                  90                  95
   Leu Lys Thr Leu Glu Pro Glu Leu Gly Thr Leu Gln Ala Arg Leu Asp
               100                 105                 110
   Arg Leu Leu Arg Arg Leu Gln Leu Leu Met Ser Arg Leu Ala Leu Pro
           115                 120                 125
   Gln Pro Pro Pro Asp Pro Pro Ala Pro Pro Leu Ala Pro Pro Ser Ser
       130                 135                 140
   Ala Trp Gly Gly Ile Arg Ala Ala His Ala Ile Leu Gly Gly Leu His
   145                 150                 155                 160
   Leu Thr Leu Asp Trp Ala Val Arg Gly Leu Leu Leu Leu Lys Thr Arg
   165 170 175
   Leu
(2) INFORMATION FOR SEQ ID NO:7:
   (i) SEQUENCE CHARACTERISTICS:
      (A) LENGTH: 507 base pairs
      (B) TYPE: nucleic acid
      (C) STRANDEDNESS: double
      (D) TOPOLOGY: linear
   (ii) MOLECULE TYPE: cDNA to mRNA
   (iii) HYPOTHETICAL: N
   (iv) ANTI-SENSE: N
   (ix) FEATURE:
      (A) NAME/KEY: CDS
      (B) LOCATION: 1..507
      (D) OTHER INFORMATION:
   (xi) SEQUENCE DESCRIPTION: SEQ ID NO:7:
   GTT TCC CCA GAC CCT CGG GCC GAG CTG GAC AGC ACC GTG CTC CTG ACC       48
   Val Ser Pro Asp Pro Arg Ala Glu Leu Asp Ser Thr Val Leu Leu Thr
     1               5                  10                  15
   CGC TCT CTC CTG GCG GAC ACG CGG CAG CTG GCT GCA CAG CTG AGG GAC       96
   Arg Ser Leu Leu Ala Asp Thr Arg Gln Leu Ala Ala Gln Leu Arg Asp
                20                  25                  30
   AAA TTC CCA GCT GAC GGG GAC CAC AAC CTG GAT TCC CTG CCC ACC CTG      144
   Lys Phe Pro Ala Asp Gly Asp His Asn Leu Asp Ser Leu Pro Thr Leu
            35                  40                  45
   GCC ATG AGT GCG GGG GCA CTG GGA GCT CTA CAG CTC CCA GGT GTG CTG      192
   Ala Met Ser Ala Gly Ala Leu Gly Ala Leu Gln Leu Pro Gly Val Leu
        50                  55                  60
   ACA AGG CTG CGA GCG GAC CTA CTG TCC TAC CTG CGG CAC GTG CAG TGG      240
   Thr Arg Leu Arg Ala Asp Leu Leu Ser Tyr Leu Arg His Val Gln Trp
    65                  70                  75                  80
   CTG CGC CGG GCA GGT GGC TCT TCC CTG AAG ACC CTG GAG CCC GAG CTG      288
   Leu Arg Arg Ala Gly Gly Ser Ser Leu Lys Thr Leu Glu Pro Glu Leu
                    85                  90                  95
   GGC ACC CTG CAG GCC CGA CTG GAC CGG CTG CTG CGC CGG CTG CAG CTC      336
   Gly Thr Leu Gln Ala Arg Leu Asp Arg Leu Leu Arg Arg Leu Gln Leu
               100                 105                 110
   CTG ATG TCC CGC CTG GCC CTG CCC CAG CCA CCC CCG GAC CCG CCG GCG      384
   Leu Met Ser Arg Leu Ala Leu Pro Gln Pro Pro Pro Asp Pro Pro Ala
           115                 120                 125
   CCC CCG CTG GCG CCC CCC TCC TCA GCC TGG GGG GGC ATC AGG GCC GCC      432
   Pro Pro Leu Ala Pro Pro Ser Ser Ala Trp Gly Gly Ile Arg Ala Ala
       130                 135                 140
   CAC GCC ATC CTG GGG GGG CTG CAC CTG ACA CTT GAC TGG GCC GTG AGG      480
   His Ala Ile Leu Gly Gly Leu His Leu Thr Leu Asp Trp Ala Val Arg
   145                 150                 155                 160
   GGA CTG CTG CTG CTG AAG ACT CGG CTG                                  507
   Gly Leu Leu Leu Leu Lys Thr Arg Leu
                   165
(2) INFORMATION FOR SEQ ID NO:8:
   (i) SEQUENCE CHARACTERISTICS:
      (A) LENGTH: 169 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Val Ser Pro Asp Pro Arg Ala Glu Leu Asp Ser Thr Val Leu Leu Thr
    1               5                  10                  15
   Arg Ser Leu Leu Ala Asp Thr Arg Gln Leu Ala Ala Gln Leu Arg Asp
               20                  25                  30
   Lys Phe Pro Ala Asp Gly Asp His Asn Leu Asp Ser Leu Pro Thr Leu
           35                  40                  45
   Ala Met Ser Ala Gly Ala Leu Gly Ala Leu Gln Leu Pro Gly Val Leu
       50                  55                  60
   Thr Arg Leu Arg Ala Asp Leu Leu Ser Tyr Leu Arg His Val Gln Trp
   65                  70                  75                  80
   Leu Arg Arg Ala Gly Gly Ser Ser Leu Lys Thr Leu Glu Pro Glu Leu
                   85                  90                  95
   Gly Thr Leu Gln Ala Arg Leu Asp Arg Leu Leu Arg Arg Leu Gln Leu
               100                 105                 110
   Leu Met Ser Arg Leu Ala Leu Pro Gln Pro Pro Pro Asp Pro Pro Ala
           115                 120                 125
   Pro Pro Leu Ala Pro Pro Ser Ser Ala Trp Gly Gly Ile Arg Ala Ala
       130                 135                 140
   His Ala Ile Leu Gly Gly Leu His Leu Thr Leu Asp Trp Ala Val Arg
   145                 150                 155                 160
   Gly Leu Leu Leu Leu Lys Thr Arg Leu
                   165
(2) INFORMATION FOR SEQ ID NO:9:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: N
   (iv) ANTI-SENSE: N
   (ix) FEATURE:
      (A) NAME/KEY: CDS
      (B) LOCATION: 1..33
      (D) OTHER INFORMATION:
   (xi) SEQUENCE DESCRIPTION: SEQ ID NO:9:
   CCA GAT ACA GCT GTC AGG CCT GGG CCA CCA CCT                           33
   Pro Asp Thr Ala Val Arg Pro Gly Pro Pro Pro
     1               5                  10
(2) INFORMATION FOR SEQ ID NO:10:
   (i) SEQUENCE CHARACTERISTICS:
      (A) LENGTH: 11 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Pro Asp Thr Ala Val Arg Pro Gly Pro Pro Pro
    1               5                   10
(2) INFORMATION FOR SEQ ID N0:11:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (iii) HYPOTHETICAL: N
   (iv) ANTI-SENSE: N
   (v) FRAGMENT TYPE: N-terminal
   (xi) SEQUENCE DESCRIPTION: SEQ ID NO:11:
(2) INFORMATION FOR SEQ ID NO:12:
   (i) SEQUENCE CHARACTERISTICS:
      (A) LENGTH: 33 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: N
   (iv) ANTI-SENSE: N
   (xi) SEQUENCE DESCRIPTION: SEQ ID NO:12:
   CCAGATACAG CTGTCGCCCC TGGGCCACCA CCT 33
(2) INFORMATION FOR SEQ ID NO:13:
   (i) SEQUENCE CHARACTERISTICS:
      (A) LENGTH: 33 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: N
   (iv) ANTI-SENSE: N
   (xi) SEQUENCE DESCRIPTION: SEQ ID NO:13:
   CCAGATACAG CTGTCAGGCC TGGGCCACCA CCT 33
(2) INFORMATION FOR SEQ ID NO:14:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: N
   (iv) ANTI-SENSE: N
   (xi) SEQUENCE DESCRIPTION: SEQ ID NO:14:
   GATCCATCGA GGGTAGGGGG CCCCCACCTG GCCCCCC 37
(2) INFORMATION FOR SEQ ID NO:15:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: N
   (iv) ANTI-SENSE: Y
   (xi) SEQUENCE DESCRIPTION: SEQ ID NO:15:
   TCGAGGGGGG CCAGGTGGGG GCCCCCTACC CTCGATG 37
(2) INFORMATION FOR SEQ ID NO:16:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: N
   (iv) ANTI-SENSE: N
   (xi) SEQUENCE DESCRIPTION: SEQ ID NO:16:
   GATACAGCTG TCGCCGGGCC CCCACCTGGC 30
(2) INFORMATION FOR SEQ ID NO:17:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (iii) HYPOTHETICAL: N
   (iv) ANTI-SENSE: N
   (v) FRAGMENT TYPE: N-terminal
   (xi) SEQUENCE DESCRIPTION: SEQ ID NO:17:
   Gly Pro Pro Pro Gly Pro
   1               5
(2) INFORMATION FOR SEQ ID NO:18:
   (i) SEQUENCE CHARACTERISTICS:
      (A) LENGTH: 33 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: N
   (iv) ANTI-SENSE: N
   (xi) SEQUENCE DESCRIPTION: SEQ ID NO:18:
   GATACAGCTG TCGCCCCTGG GCCACCACCT GGC 33
(2) INFORMATION FOR SEQ ID NO:19:
   (i) SEQUENCE CHARACTERISTICS:
      (A) LENGTH: 30 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: N
   (iv) ANTI-SENSE: N
   (xi) SEQUENCE DESCRIPTION: SEQ ID NO:19:
   GATACAGCTG TCGCCGGGCC CCCACCTGGC 30